(19) Europäisches Patentamt
European Patent Office
Office européen des brevets

(11) **EP 4 142 822 B1**

(12) **EUROPÄISCHE PATENTSCHRIFT**

(45) Veröffentlichungstag und Bekanntmachung des Hinweises auf die Patenterteilung:
**18.03.2026   Patentblatt 2026/12**

(21) Anmeldenummer: **21721429.5**

(22) Anmeldetag: **21.04.2021**

(51) Internationale Patentklassifikation (IPC):
*A61M 1/16* (2006.01)

(52) Gemeinsame Patentklassifikation (CPC):
**A61M 1/1617**

(86) Internationale Anmeldenummer:
**PCT/EP2021/060306**

(87) Internationale Veröffentlichungsnummer:
**WO 2021/219448 (04.11.2021 Gazette 2021/44)**

(54) **DIALYSEVORRICHTUNG MIT EINER VORRICHTUNG ZUR BESTIMMUNG VON MINDESTENS ZWEI HÄMODIALYSEPARAMETERN**

DIALYSIS DEVICE WITH A DEVICE FOR DETERMINING AT LEAST TWO HEMODIALYSIS PARAMETRES

DISPOSITIF DE DIALYSE AVEC UN DISPOSITIF POUR DÉTERMINER AU MOINS DEUX PARAMÈTRES DE L'HÉMODIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.04.2020   DE 102020111358**

(43) Veröffentlichungstag der Anmeldung:
**08.03.2023   Patentblatt 2023/10**

(73) Patentinhaber: **Fresenius Medical Care Deutschland GmbH**
**61352 Bad Homburg v.d.H (DE)**

(72) Erfinder: **MAIERHOFER, Andreas**
**97422 Schweinfurt (DE)**

(74) Vertreter: **Oppermann, Frank**
**OANDO Oppermann & Oppermann**
**Thurn-und-Taxis-Platz 6**
**60313 Frankfurt am Main (DE)**

(56) Entgegenhaltungen:
**US-A1- 2005 148 923**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

# EP 4 142 822 B1

## Beschreibung

Die Erfindung betrifft eine Dialysevorrichtung mit einer Vorrichtung zur Bestimmung von mindestens einem ersten und zweiten Hämodialyseparameter. Darüber hinaus betrifft die Erfindung ein Dialysesystem mit einer Dialysevorrichtung, die einen Dialysator und einen extrakorporalen Blutkreislauf umfasst, und einer Vorrichtung zur Bestimmung von mindestens einem ersten und zweiten Hämodialyseparameter. Des Weiteren betrifft die Erfindung ein Computerprogrammprodukt, das Befehle umfasst, die bei der Ausführung des Programms durch einen Computer diesen veranlassen, ein Verfahren zur Bestimmung von mindestens einem ersten und zweiten Hämodialyseparameter während einer Dialysebehandlung mit einer Dialysevorrichtung auszuführen.

Bei Verfahren der chronischen Blutreinigungstherapie wie Hämodialyse, Hämofiltration und Hämodiafiltration wird Blut über einen extrakorporalen Blutkreislauf durch eine Blutbehandlungseinheit, beispielsweise einen Dialysator bzw. Filter geleitet. Nachfolgend werden Dialysator und Filter als Synonyme verstanden. Als Zugang zum Blutgefäßsystem wird häufig operativ eine arteriovenöse Fistel angelegt, die im Allgemeinen mit einer arteriellen und venösen Kanüle punktiert wird. Ebenso ist der Einsatz eines Gefäßimplantats möglich. Wenn nachfolgend von dem Begriff "Gefäßzugang" die Rede ist, wird darunter jede Art eines Zugangs zum Blutgefäßsystem eines Patienten verstanden. Insbesondere werden unter einem Gefäßzugang die durch die Verbindung zwischen einer Arterie und einer Vene des Patienten entstehenden vergrößerten Blutgefäße verstanden. Bei dem Gefäß kann es sich auch um ein durch einen Graft erzeugtes Gefäß handeln.

Zur Überwachung einer Dialysebehandlung sind verschiedene Parameter von Interesse, die nachfolgend als Hämodialyseparamter bezeichnet werden.

Ein für die Effizienz einer Dialyse relevanter Hämodialyseparamter ist die Dialysedosis Kt/V, die eine dimensionslose Größe ist. Die Dialysedosis ist der Quotient aus dem Produkt von Harnstoff-Clearance K (ml/min) und Dialysedauer t (min) und dem Harnstoff-Verteilungsvolumen V. Die Clearance ist diejenige Blutplasmamenge, die pro Zeiteinheit beim Durchfluss durch die Nieren bzw. die Membran eines Dialysators vollständig von einer bestimmten Substanz gereinigt wird. Die "Dialysance" ist in der Dialyse-Fachliteratur ein üblicher Begriff (z. B. Petitclerc, Gotch, ...), wenn es um die Annäherung an eine Referenzkonzentration geht, z. B. Natrium, d. h. wenn der betrachtete Stoff auf beiden Seiten der Membran vorhanden ist. Eine on-line Bestimmung der ionischen Dialysance kann erfolgen, um die Clearance kleiner Moleküle, wie Urea zu bestimmen. Zwischen den Begriffen Clearance K und Dialysance D besteht der nachfolgend erläuterte Zusammenhang.

Für den Mediziner ist die Clearance K "der Teilstrom (in ml/min) des Blutes, der vollständig von der betrachteten Substanz befreit wird". Mathematisch gesehen ist aber die Beschränkung auf den Fall, dass der Transport in einen Raum erfolgt, in dem der Stoff nicht vorhanden ist, nicht nötig. Die zugrundeliegende Kinetik (1-Pool-Modell) wird beschrieben durch $V * dc_b/dt = - K (c_b - c_d)$, wobei V: Verteilungsvolumen, dc: Veränderung einer Konzentration, b: Blut, d: Dialysat; K: "Transmembranfluss", "Reaktionskinetik", ...; und hängt direkt von den Membraneigenschaften und der Diffusionskonstanten des betrachteten Stoffs ab, mit den Einheiten [K]= ml/min, [c]= Anzahl/ ml, V: Verteilungsvolumen. Bei $c_d$ = 0 würde der Begriff Clearance verwendet werden. Für $c_d > 0$ beschreibt K noch immer die Kinetik korrekt, aber die anschauliche Definition ist dann "Teilstrom, der vollständig auf die Konzentration des Dialysats gebracht wird". Dafür wird der Begriff "Dialysance" verwendet. Im Bereich der Dialyse wird der Begriff "Clearance" in der Regel Substanzen zugeordnet, die nicht im Dialysat vorhanden sind (beispielsweise Harnstoff, Creatinin, Beta-2-M, Antibiotika), und "Dialysance" Stoffen, die auch im Dialysat vorliegen (beispielsweise Na). Zahlenmäßig wäre eine Natrium-Dialysance immer gleich einer Natrium-Clearance, wobei die Natrium-Clearance dann verwendet würde, wenn gegen RO-Wasser dialysiert würde. Die Clearance K und die Dialysance D im Sinne dieser Beschreibung ist synonym zu verstehen.

Zur Bestimmung der Clearance K (Dialysance) gehören verschiedene Verfahren zum Stand der Technik. Weit verbreitet ist die Messung der ionischen Dialysance mit dem sogenannten Online Clearance Monitoring OCM (Fresenius Medical Care), das die indirekte Bestimmung der Harnstoff-Clearance während der Dialyse erlaubt. Da die Behandlungsdauer (t) bekannt ist, kann das System nach Eingabe des Harnstoff-Verteilungsvolumens (V) die Dialysedosis Kt/V online während der Dialyse ermitteln.

Die online Bestimmung der Clearance beruht auf der zeitlichen Veränderung einer physikalischen oder chemischen Kenngröße der Dialysierflüssigkeit im Dialysierflüssigkeitssystem. Eine physikalische oder chemische Kenngröße der Dialysierflüssigkeit wird stromauf des Dialysators verändert und die auf die zeitliche Veränderung der physikalischen oder chemischen Kenngröße der Dialysierflüssigkeit stromauf des Dialysators zurückzuführende zeitliche Veränderung der physikalischen oder chemischen Kenngröße der Dialysierflüssigkeit stromab des Dialysators wird erfasst. Die zeitliche Veränderung der physikalischen oder chemischen Kenngröße der Dialysierflüssigkeit im Dialysierflüssigkeitssystem wird nachfolgend als Bolus bezeichnet.

Die EP 0 911 043 B1 beschreibt eine Dialysevorrichtung, die über eine Rechen- und Auswerteeinheit verfügt, die derart ausgebildet ist, dass die Dialysance D aus der stromauf des Dialysators als Bolus der Dialysierflüssigkeit zugegebenen Menge eines Stoffes und dem Integral über die Zeit der auf den Bolus zurückzuführenden Veränderung der Konzentration eines Stoffes in der Dialysierflüssigkeit stromab des Dialysators sowie dem Dialysierflüssigkeitsfluss bestimmt wird. Ein Bolus kann beispielsweise durch eine Veränderung der Natriumionenkonzentration verabreicht werden, beispielsweise kann eine NaCl-enthaltende Lösung zudosiert werden. Das Zudosieren kann online mit einer Variation des Dosiervolumens eines Konzentrats erfolgen. Die Änderung kann erfolgen, indem mehr oder weniger Konzentrat zudosiert wird (im Vergleich zur Basisdosierung, die zu der Basislinie führt). Dadurch sind Änderungen in Form eines positiven Bolus oder eines negativen Bolus in beide Richtungen möglich. Anzumerken ist hier, dass keine reine NaCl-Lösung als Konzentrat zudosiert werden muss, sondern auch eine Lösung verschiedener Ionen zudosiert werden kann. Für die Erzeugung des Bolus geht es im Allgemeinen nur um die Leitfähigkeit, d. h. die Form des Bolus, die durch die Fläche und/oder Amplitude und/oder Dauer des Bolus beschrieben werden kann, wird durch die Konzentrationsänderung aller im Dialysat enthaltenen Ionen beeinflusst.

Ein weiterer für die Dialysebehandlung wichtiger Hämodialyseparameter ist der "blood access flow", der nachfolgend als Blutfluss im Gefäßzugang Qa bezeichnet wird. Zum Stand der Technik gehören verschiedene Verfahren zur Bestimmung von Qa. Es sind Verfahren zur Bestimmung von Qa bekannt, die auf der Bestimmung der Clearance bei unterschiedlichen Flussrichtungen des Blutes beruhen.

Die EP 0 928 614 B1 beschreibt eine Dialysevorrichtung, die über eine Einrichtung zur Erfassung einer auf den Bolus zurückzuführenden zeitlichen Veränderung der physikalischen oder chemischen Kenngröße der Dialysierflüssigkeit im Dialysierflüssigkeitssystem für zwei unterschiedliche Dialysebedingungen verfügt. Unter der ersten Dialysebedingung fördert die erste Blutleitung Blut von einem stromabwärts gelegenen Teil eines Gefäßzugangs des Patienten in den Dialysator und die zweite Blutleitung fördert Blut von dem Dialysator in Richtung auf einen stromaufwärts gelegenen Teil des Gefäßzugangs, und unter der zweiten Dialysebedingung fördert die erste Blutleitung Blut von einem stromaufwärts gelegenen Teil des Gefäßzugangs und die zweite Blutleitung fördert Blut in Richtung auf einen stromabwärts gelegenen Teil des Gefäßzugangs. Das bekannte Verfahren zur Bestimmung von Qa beruht darauf, einen Wert für die Dialysance für die erste Dialysebedingung und einen Wert für die Dialysance für die zweite Dialysebedingung zu ermitteln und aus den beiden Werten für die Dialysance den Blutfluss im Gefäßzugang Qa zu berechnen. Die Ermittlung der Clearance (Dialysance) kann für die unterschiedlichen Dialysebedingungen mit den bekannten Verfahren online erfolgen (Online Clearance Monitoring OCM).

Aus der US 2005/0148923 A1 ist eine Dialysevorrichtung bekannt, die über eine Vorrichtung zur Bestimmung eines Hämodialyseparameters verfügt. Die Dialysevorrichtung weist einen Dialysator auf, der durch eine semipermeable Membran in ein erstes Kompartiment, das Teil eines extrakorporalen Blutkreislaufs ist, und ein zweites Kompartiment, das Teil eines Dialysierflüssigkeitssystems ist, unterteilt ist. Die Bestimmung des Hämodialyseparamters beruht auf der Erzeugung einer messbaren Änderung der Konzentration einer Substanz in dem Blutkreislauf, wobei die Konzentration dieser Substanz im Dialysierflüssigkeitssystem am Auslass des Dialysators erfasst wird und der Dialyseparameter auf der Grundlage der integrierten Konzentration bestimmt wird.

Der Erfindung liegt die Aufgabe zugrunde, mindestens zwei Hämodialyseparameter während einer Dialysebehandlung mit möglichst großer Genauigkeit bei einer möglichst geringen Belastung für den Patienten zu bestimmen.

Die Lösung dieser Aufgabe erfolgt erfindungsgemäß mit den Merkmalen der unabhängigen Patentansprüche. Die abhängigen Ansprüche betreffen bevorzugte Ausführungsformen der Erfindung.

Die erfindungsgemäße Dialysevorrichtung verfügt über eine Vorrichtung zur Bestimmung von mindestens einem ersten und zweiten Hämodialyseparameter. Die Dialysevorrichtung ist dafür eingerichtet, mit einem Dialysator verbunden zu werden, der durch eine semipermeable Membran in ein erstes Kompartiment, das Teil eines extrakorporalen Blutkreislaufs ist, und ein zweites Kompartiment, das Teil eines Dialysierflüssigkeitssystems, unterteilt ist. Die Dialysevorrichtung ist weiterhin dafür eingerichtet mit einer ersten und einer zweite Blutleitung verbunden zu werden, die mit einem Einlass bzw. Auslass des ersten Kompartiments des Dialysators verbunden werden. Die Blutleitungen (Blutschlauchsystem) sind Teil des extrakorporalen Blutkreislaufs.

Die Vorrichtung zur Bestimmung von Hämodialyseparametern umfasst eine Einrichtung zur Erzeugung einer zeitlichen Veränderung einer physikalischen oder chemischen Kenngröße von im Dialysierflüssigkeitssystem befindlicher Dialysierflüssigkeit in einer Dialysierflüssigkeitszuführleitung zu dem Dialysator in Form eines Bolus und eine Einrichtung zur Erfassung einer auf den Bolus zurückzuführenden zeitlichen Veränderung der physikalischen oder chemischen Kenngröße der Dialysierflüssigkeit in einer Dialysierflüssigkeitsabführleitung aus dem Dialysator. Die Veränderung der physikalischen oder chemischen Kenngröße kann stromauf des zweiten Kompartiments des Dialysators gemessen werden und die auf die Veränderung der Kenngröße zurückzuführende Veränderung der Kenngröße kann stromab des zweiten Kompartiments des Dialysators gemessen werden.

Die Veränderung der Kenngröße kann für eine erste Dialysebedingung, unter der die erste Blutleitung Blut von einem stromabwärts gelegenen Teil eines Gefäßzugangs des Patienten fördert und die zweite Blutleitung Blut in Richtung auf einen stromaufwärts gelegenen Teil des Gefäßzugangs fördert und/oder für eine zweite Dialysebedingung, bei der die erste Blutleitung Blut von einem stromaufwärts gelegenen Teil des Gefäßzugangs fördert und die zweite Blutleitung Blut in Richtung auf einen stromabwärts gelegenen Teil des Gefäßzugangs fördert, erfolgen.

Physikalische oder chemische Kenngrößen können unterschiedliche Eigenschaften der Dialysierflüssigkeit sein, die sich im Dialysierflüssigkeitssystem messen lassen. Diese Eigenschaften können direkt gemessen werden oder mit diesen Eigenschaften korrelierende Größen können gemessen werden. Beispielsweise kann die Kenngröße die Konzentration eines Stoffes in der Dialysierflüssigkeit (Stoffkonzentration), beispielsweise die Konzentration eines oder mehrerer Elektrolyte, insbesondere die Na-Konzentration sein. Beispielsweise können die Leitfähigkeit oder optische Eigenschaften der Dialysierflüssigkeit, beispielsweise die Absorbanz oder der Drehwinkel des Lichts (Glukose) oder die Ausbreitungsgeschwindigkeit von Ultraschall gemessen werden. Alternativ können für die Messung auch chemische Sensoren eingesetzt werden.

Darüber hinaus verfügt die erfindungsgemäße Dialysevorrichtung über eine mit der Einrichtung zur Erzeugung einer Veränderung einer physikalischen oder chemischen Kenngröße und der Einrichtung zur Erfassung einer zeitlichen Veränderung einer physikalischen oder chemischen Kenngröße zusammenwirkenden Rechen- und Auswerteeinheit, die derart konfiguriert ist, dass auf der Grundlage der Veränderung der physikalischen oder chemischen Kenngröße in Form eines Bolus mindestens ein Wert für die Clearance der Dialysebehandlung bestimmt wird, und auf der Grundlage des mindestens einen Wertes für die Clearance mindestens ein Hämodialyseparameter bestimmt wird.

Der Erfinder hat erkannt, dass die Art der zeitlichen Veränderung der Kenngröße für die Bestimmung einzelner Hämodialyseparameter, d. h. die Form des Bolus, zum einen unter dem Gesichtspunkt einer möglichst genauen Messung und zum anderen unter Berücksichtigung einer möglichst geringen Belastung für den Patienten entscheidend sein kann. Es hat sich insbesondere herausgestellt, dass ein für eine Clearance-Messung zur Bestimmung der Dialysedosis Kt/V verabreichter Bolus nicht in gleichem Maße für eine Clearance-Messung zur Bestimmung des Blutflusses im Gefäßzugang Qb mit hinreichender Genauigkeit geeignet ist.

Die erfindungsgemäße Dialysevorrichtung berücksichtigt auch, dass sich in der Praxis gezeigt hat, dass für die Bestimmung von Hämodialyseparametern, insbesondere zur Bestimmung der Dialysedosis Kt/V oder des Blutflusses im Gefäßzugang Qa, verschiedene Randbedingungen einzuhalten sind.

Die erfindungsgemäße Dialysevorrichtung zeichnet sich dadurch aus, dass für die unterschiedlichen Messungen unterschiedliche Boli verabreicht werden. Für die Bestimmung des ersten Hämodialyseparameters wird ein "großer Bolus" verabreicht, während für die Bestimmung des zweiten Hämodialyseparameters ein "kleiner Bolus" verabreicht wird. Ein kleiner Bolus hat grundsätzlich den Vorteil einer geringeren Belastung für den Patienten, während ein großer Bolus grundsätzlich den Vorteil einer großen Messgenauigkeit hat.

Die Einrichtung zur Erzeugung einer Veränderung einer physikalischen oder chemischen Kenngröße ist derart ausgebildet,

dass zur Bestimmung eines ersten Hämodialyseparameters mindestens ein Bolus für die Bestimmung des ersten Hämodialyseparameters mit einer ersten Größe (A) erzeugt wird, wobei die Rechen- und Auswerteeinheit derart konfiguriert ist, dass auf der Grundlage der Veränderung der physikalischen oder chemischen Kenngröße in Form mindestens eines Bolus für die Bestimmung des ersten Hämodialyseparameters mindestens ein Wert für die Clearance der Dialysebehandlung bestimmt wird, auf dessen bzw. auf deren Grundlage der erste Hämodialyseparameter bestimmt wird, und

dass zur Bestimmung eines zweiten Hämodialyseparameters mindestens ein Bolus für die Bestimmung des zweiten Hämodialyseparameters mit einer zweiten Größe (A') erzeugt wird, wobei die Rechen- und Auswerteeinheit derart konfiguriert ist, dass auf der Grundlage der Veränderung der physikalischen oder chemischen Kenngröße in Form mindestens eines Bolus für die Bestimmung des zweiten Hämodialyseparameters mindestens ein Wert für die Clearance der Dialysebehandlung bestimmt wird, auf dessen bzw. auf deren Grundlage der zweite Hämodialyseparameter bestimmt wird.

Ein erster Aspekt der Erfindung sieht vor, dass die erste Größe (A) größer als die zweite Größe (A') ist, während ein zweiter Aspekt vorsieht, dass der Abstand der ersten Größe (A) zu einer Bezugsgröße größer als der Abstand der zweiten Größe (A') zu einer Bezugsgröße ist.

Die Größe des Bolus wird durch dessen Form bestimmt. Für die Form des Bolus kann ein Integral (Fläche) oder die Amplitude und/oder Dauer des Bolus charakteristisch sein. Der zeitliche Verlauf der Änderung braucht nicht exakt beschrieben zu werden, sondern kann auch angenähert werden.

Der Quotient zwischen dem ersten Flächeninhalt (A) und zweiten Flächeninhalt (A') ist insbesondere größer als 2,5, insbesondere größer als 2,7, insbesondere größer als 3.

Eine Ausführungsform gemäß dem ersten Aspekt der Erfindung sieht vor, dass der mindestens eine Bolus für die Bestimmung des ersten Hämodialyseparameters durch eine erste Fläche und der mindestens eine Bolus für die Bestimmung des zweiten Hämodialyseparameters durch eine zweite Fläche charakterisiert ist, wobei der Flächeninhalt der ersten Fläche (erster Flächeninhalt) größer als der Flächeninhalt der zweiten Fläche (zweite Flächeninhalt) ist. Folglich kann mit einem "großen Bolus" der erste Hämodialyseparameter grundsätzlich mit einer höheren Genauigkeit bestimmt werden, was aber dazu führen könnte, dass die Bestimmung dieses Parameters mit einer höheren Belastung für den Patienten verbunden ist. Der zweite Hämodialyseparameter kann mit einem "kleinen Bolus" zwar grundsätzlich nur mit einer geringeren Genauigkeit bestimmt werden, eine möglichweise auftretende Belastung für den Patienten ist aber ausgeschlossen. In diesem Zusammenhang wird unter dem Flächeninhalt des Bolus der Flächeninhalt der Fläche verstanden, die zwischen dem Graphen einer die zeitliche Veränderung der physikalischen oder chemischen Kenngröße beschreibenden Funktion und dem Graphen einer Referenzfunktion liegt.

Die Referenzfunktion kann eine Basislinie sein, die durch einen Verlauf der physikalischen oder chemischen Kenngröße vor und/oder nach dem Bolus ermittelt werden kann. Bei der Basislinie kann es sich um einen gerade Linie, eine Kurve oder eine andere von einer geraden Linie abweichende Funktion handeln. Die geradlinige Basislinie kann keine Steigung aufweisen oder eine Steigung aufweisen. Die Basislinie kann den Verlauf der Kenngröße bereinigt um Rauschsignale oder Schwankungen beschreiben.

Die Messgenauigkeit kann zwar grundsätzlich auch dadurch verbessert werden, dass mehrere Messungen vorgenommen und die Messergebnisse statistisch ausgewertet werden. Dieser Ansatz ist aber mit dem Nachteil verbunden, dass die Bestimmung des Hämodialyseparameters relativ viel Zeit in Anspruch nimmt und der Patient aufgrund der mehrfachen Messungen stärker belastet werden könnte.

Gemäß dem zweiten Aspekt der Erfindung wird eine Bezugsgröße definiert, wobei die Größe des mindestens einen Bolus für die Bestimmung des ersten Hämodialyseparameters und die Größe des mindestens einen Bolus für die Bestimmung des zweiten Hämodialyseparameters auf die Bezugsgröße bezogen werden. Die Bezugsgröße kann das Rauschen im System sein, wobei der Abstand zu der Bezugsgröße ein Signal-Rausch-Abstand sein kann. Für eine genaue Messung wird grundsätzlich ein möglichst großer Signal-Rausch-Abstand angestrebt, der mit zunehmender Größe des Bolus zunimmt, womit aber auch die Belastung für den Patienten zunimmt. In Analogie zu dem ersten Aspekt wird bei dem zweiten Aspekt der Erfindung für die Bestimmung des ersten Hämodialyseparameters ein größerer Signal-Rausch-Abstand als für die Bestimmung des zweiten Hämodialyseparameters angestrebt.

Die Vorteile der Erfindung kommen insbesondere zum Tragen, wenn der erste Hämodialyseparameter der Blutfluss im Gefäßzugang Qb oder eines Rezirkulationsflusses in einem Gefäßsystem und der zweite Hämodialyseparameter die Dialysedosis Kt/V ist.

Zur Bestimmung des Blutflusses im Gefäßzugang kann die Einrichtung zur Erzeugung einer Veränderung einer physikalischen oder chemischen Kenngröße derart ausgebildet sein, dass für eine erste Dialysebedingung ein erster Bolus mit dem ersten Flächeninhalt (A) und für die zweite Dialysebedingung ein zweiter Bolus mit dem ersten Flächeninhalt (A) erzeugt wird, wobei die Rechen- und Auswerteeinheit, derart konfiguriert ist, dass auf der Grundlage der Veränderung der physikalischen oder chemischen Kenngröße in Form des ersten Bolus mit dem ersten Flächeninhalt (A) unter der ersten Dialysebedingung ein erster Wert für die Clearance und auf der Grundlage der Veränderung der physikalischen oder chemischen Kenngröße in Form des zweiten Bolus unter der zweiten Dialysebedingung ein zweiter Wert für die Clearance bestimmt wird, auf deren Grundlage der Blutfluss im Gefäßzugang bestimmt wird.

Unter der ersten Dialysebedingung fördert die erste Blutleitung Blut von einem stromabwärts gelegenen Teil eines Gefäßzugangs des Patienten und die zweite Blutleitung fördert Blut in Richtung auf einen stromaufwärts gelegenen Teil des Gefäßzugangs und unter der zweiten Dialysebedingung fördert die erste Blutleitung Blut von einem stromaufwärts gelegenen Teil des Gefäßzugangs und die zweite Blutleitung fördert Blut in Richtung auf einen stromabwärts gelegenen Teil des Gefäßzugangs.

Mit anderen Worten umfasst die Dialysebedingung die Nadelorientierung, die beispielsweise als normale Orientierung oder invertierte Orientierung bezeichnet werden kann. Unter Änderung der Nadelorientierung bzw. erster und zweiter Dialysebedingung ist auch zu verstehen, dass die Nadeln ausgetauscht werden oder der Fluss durch die Nadeln umgekehrt wird. Dies kann beispielsweise dadurch erreicht werden, dass die Blutpumpe in umgekehrter Richtung fördert oder dass eine Flussumkehreinrichtung vorgesehen ist, mit der die bei normaler Behandlung venöse Nadel mit dem arteriellen Blutschlauch (oder einem Abschnitt davon) und die bei normaler Behandlung arterielle Nadel mit dem venösen Blutschlauch (oder einem Abschnitt davon) verbunden wird. Diese Flussumkehreinrichtung kann manuell zu bedienen sein oder automatisch ansteuerbar sein. Ein Beispiel für eine manuell zu bedienende Flussumkehreinrichtung ist der sogenannte Twister® der Firma Fresenius Medical Care Deutschland GmbH.

Bei den beiden Dialysebedingungen sind vorzugsweise alle anderen Parameter, die einen Einfluss auf die Clearance haben, während der Messungen gleich oder weitestgehend gleich. Sind diese anderen Parameter nicht gleich, so kann die Dialysevorrichtung derart ausgebildet sein, dass bei der Bestimmung des Blutflusses im Gefäßzugang oder eines Rezirkulationsflusses in einem Gefäßsystem, die bei bestimmten Clearance-Werte um den Einfluss dieser Unterschiede rechnerisch korrigiert unterschiedlichen anderen Parametern werden. Beispielsweise kann ein erhöhter Dialysierflüssigkeitsfluss mittels einer hinterlegten Abhängigkeit der Clearance vom Dialysierflüssigkeitsfluss korrigiert werden. Diese Abhängigkeit kann in Form einer Tabelle oder einer Gleichung bzw. eines Algorithmus hinterlegt werden.

Eine alternative Ausführungsform sieht vor, dass die Dialysevorrichtung derart ausgebildet ist, dass bei der ersten Dialysebedingung ein Clearance-Wert nicht gemessen, sondern abgeschätzt wird, und bei der zweiten Dialysebedingung ein Clearance-Wert mittels des oben beschriebenen Verfahrens unter Veränderung einer physikalischen oder chemischen Kenngröße gemessen wird. Der Blutfluss im Gefäßzugang Qa wird dann unter Verwendung des geschätzten Clearance-Wertes und des gemessenen Clearance-Wertes bestimmt.

Die Abschätzung der Clearance anstelle einer Messung ist im Allgemeinen nur für die Bestimmung des ersten Dialyseparameters bei der ersten Dialysebedingung, insbesondere des Blutflusses im Gefäßzugang, von ausreichender Genauigkeit, da bei der ersten Dialysebedingung eine Fistelrezirkulation nicht zu erwarten ist oder zumindest gering ist. Bei der zweiten Dialysebedingung tritt hingegen eine Fistelrezirkulation auf. Daher macht eine Abschätzung auch des zweiten Dialyseparameters bei der zweiten Dialysebedingung keinen Sinn.

Die alternative Ausführungsform mit einer Abschätzung der Clearance anstelle einer Messung für die Bestimmung des ersten Clearance-Wertes unter der ersten Dialysebedingung zur Bestimmung des ersten Dialyseparameters, insbesondere des Blutflusses im Gefäßzugang, ist von eigener erfinderischer Bedeutung, d. h. die Bestimmung des ersten Dialyseparameters mit diesem Verfahren kann unabhängig von der Bestimmung eines zweiten Dialyseparameters erfolgen und unabhängig von dem erfindungsgemäßen Prinzip der Bemessung von Boli unterschiedlicher Größe.

Bei der alternativen Ausführungsform ist zur Bestimmung des Blutflusses im Gefäßzugang Qa die Einrichtung zur Erzeugung einer Veränderung einer physikalischen oder chemischen Kenngröße derart ausgebildet, dass für eine zweite Dialysebedingung ein Bolus mit dem ersten Flächeninhalt (A) erzeugt wird, wobei die Rechen- und Auswerteeinheit, derart konfiguriert ist, dass ein erster Wert für die Clearance auf der Grundlage einer Schätzung der Clearance für die erste Dialysebedingung und auf der Grundlage der Veränderung der physikalischen oder chemischen Kenngröße in Form des Bolus mit dem ersten Flächeninhalt (A) unter der zweiten Dialysebedingung ein zweiter Wert für die Clearance bestimmt wird, auf deren Grundlage der Blutfluss im Gefäßzugang bestimmt wird.

Wenn die Bestimmung des ersten Hämodialyseparameters, insbesondere des Blutflusses im Gefäßzugang Qa, nach dem oben beschriebenen Verfahren auf der Grundlage einer Abschätzung des ersten Clearance-Wertes anstelle einer Messung des Clearance-Wertes mit einem Bolus, erfolgt, kann die Dialysevorrichtung optional ferner ausgebildet sein, einzelne Schritte zur Bestimmung von Qa nur dann durchzuführen, wenn bestimmte Bedingungen erfüllt sind, die auf eine ausreichende Genauigkeit schließen lassen.

Die Dialysevorrichtung kann derart eingerichtet sein, dass nach der Schätzung des ersten Clearance-Wertes die Messung des zweiten Clearance-Wertes nur dann ausgeführt wird, wenn eine vorbestimmte Bedingung für die Schätzung des ersten Clearance-Wertes erfüllt ist, beispielsweise wenn der geschätzte Clearance-Wert nicht bestimmten Plausibilitätskriterien genügt, beispielsweise die Abweichung des geschätzten Clearance-Wertes von einem vorgegebenen Wert zu groß ist.

Wenn die Abweichung zu groß ist, kann der erste Clearance-Werte nach Verabreichen eines Bolus gemessen werden, um einen Wert mit einer ausreichenden Genauigkeit zu haben. Dann kann die Messung des zweiten Clearance-Wertes erfolgen.

Anstelle der Überprüfung des geschätzten ersten Clearance-Wertes kann auch der nach der Schätzung des ersten Clearance-Wertes und der Messung des zweiten Clearance-Wertes der aus dem ersten und zweiten Wert ermittelte Blutfluss im Gefäßzugang Qa überprüft werden. Wenn der Blutfluss im Gefäßzugang Qa nicht sinnvoll ist oder zu stark von einem zu erwartenden ermittelten Blutflusswert im Gefäßzugang abweicht, kann die Ermittlung des Blutflusses auf der Grundlage von zwei aufeinanderfolgenden Messungen mit einem ersten und zweiten Bolus erfolgen. Hierzu kann die Dialysevorrichtung derart ausgebildet sein, dass dann noch eine Messung des ersten Clearance-Wertes durchgeführt wird.

Die Schätzung der Clearance kann beispielsweise anhand der Gleichungen erfolgen, die in Sargent, A. & Gotch, F., "Principles and biophysics of Dialysis" in Replacement of renewal function by dialysis", 4th ED, veröffentlicht sind. Zur Ermittlung eines Schätzwerts für die Clearance können bestimmte Parameter des Dialysators im Labor ermittelt oder Herstellerangaben, beispielsweise in Form einer auf dem Dialysator vorhandenen Kodierung (Barcode) oder einer aufgedruckten Zahl, die vom Anwender zu übertragen ist, oder für den Nutzer im Dialysegerät durch Auswahl eines Dialysatortyps hinterlegten Parameters, herangezogen werden. Ferner können Blutfluss, Dialysatfluss, Ultrafiltrationsfluss über die Dialysatormembran und/oder Substituatfluss berücksichtigt werden. Die herangezogene Gleichung für die Clearance bzw. Dialysance kann lauten (ohne Ultrafiltrationsfluss oder Substituatfluss):

$$D = Q_b \, \frac{e^{\gamma}-1}{e^{\gamma}-\dfrac{Q_b}{Q_d}}, \quad \gamma = k_0 A \frac{Q_d-Q_b}{Q_b \, Q_d}$$

$k_o$A Massentransferkoeffizient-Membranflächenprodukt
$Q_b$ ist der durch den Dialysator fließende Blutfluss
$Q_d$ ist der effiziente durch den Dialysator fließende Dialysatfluss.

Die Schätzung der Clearance kann auch nach dem in der EP 1 698 360 B1 beschriebenen Verfahren erfolgen. In der EP 1 698 360 B1 ist das folgende Verfahren zur Abschätzung der Clearance offenbart:

Die Clearance K, die sich während der Therapie zeitabhängig ändert, berechnet sich nach der folgenden Gleichung:

$$K(t) = K_L * C(t)$$

K ist der sich mit der Therapiezeit t ändernde Wert der Clearance.

$K_L$ ist die unter Laborbedingungen ermittelte Clearance des betreffenden Typs von Dialysator.

C(t) ist ein Korrekturfaktor, der die Verminderung der Filterleistung $K_L$ über die Therapiezeit z. B. durch die Bildung einer Sekundärmembran beschreibt.

C ist kleiner oder gleich 1, C(t) = 1-($X_1$ * t), wobei $X_1$ ein empirisch ermittelter Patienten/Filterfaktor ist.

$K_L$ ist eine Funktion von mehreren Geräteparametern:

$$K_L = f (Q_b; Q_d; Q_u; FT)$$

$Q_b$ ist der durch den Dialysator fließende Blutfluss.

$Q_d$ ist der effiziente durch den Dialysator fließende Dialysatfluss.

$Q_U$ ist der über die Membran fließende Ultrafiltrationsfluss.

FT ist der Filtertyp.

$K_L$ wird in der Regel aus Tabellen oder dreidimensionalen Matrizen ermittelt, deren Werte unter Laborbedingungen gemessen wurden, und die in den Gebrauchsanweisungen der unterschiedlichen Dialysatoren angegeben sind.

Wenn der erste Clearance-Wert bei der ersten Dialysebedingung geschätzt oder gemessen wird und der zweite Clearance-Wert bei der zweiten Dialysebedingung gemessen wird, kann aus dem ersten und zweiten Clearance-Wert der erste Dialyseparameter, insbesondere der Blutfluss im Gefäßzugang Qa, berechnet werden.

Der Blutfluss im Gefäßzugang Qa kann aus der in der nachfolgenden Figurenbeschreibung angegebenen Gleichung (4) berechnet werden.

Die Bestimmung des Blutflusses im Gefäßzugang Qa ist alternativ nach der folgenden Gleichung möglich:

$$Qa = (1/R-1)*Qb$$

Qa ist der Blutfluss im Gefäßzugang

R ist die Fistelrezirkulation (bei vertauschter Nadelposition)

Qb ist der durch den Dialysator fließende Blutfluss.

Zur Bestimmung des Blutflusses im Gefäßzugang Qa nach der obigen Gleichung muss zunächst die Rezirkulation im Gefäßzugang (Fistelrezirkulation) R bestimmt werden. Die Rezirkulation im Gefäßzugang kann auch abgeschätzt werden. Ein Schätzwert für eine Rezirkulation im Gefäßzugang kann aus gemessener Clearance und geschätzter Clearance bei den unterschiedlichen Dialysebedingungen, beispielsweise aus dem Verhältnis aus gemessener Clearance und geschätzter Clearance, ermittelt werden, wobei die Abschätzung darin besteht, die kardiopulmonare Rezirkulation zu berücksichtigen.

Ein Verfahren zur Bestimmung des Blutflusswertes im Gefäßzugang unter Abschätzung der Clearance kann somit folgende Schritte beinhalten:

1. Ermittlung eines Schätzwerts für die Clearance basierend auf Dialysatorparametern (z. B. Herstellerdaten, Barcode, etc.);

2. Änderung der Dialysebedingung, insbesondere Inversion der Nadelorientierung;

3. Messung der Clearance bei der geänderten Dialysebedingung bzw. in vertauschter Nadelposition;

4. Bestimmung des Blutflusswerts im Gefäßzugang Qa unter Verwendung der geschätzten Clearance und der gemessenen Clearance, wobei der Schritt 4 die Schritte 4a1. und 4b1. oder den Schritt 4a2. umfassen kann;

4a1. Ermittlung eines Schätzwerts für eine Rezirkulation im Gefäßzugangs aus gemessener Clearance und geschätzter Clearance, beispielsweise aus dem Verhältnis aus gemessener Clearance und geschätzter Clearance, wobei die Abschätzung darin besteht, die kardiopulmonare Rezirkulation zu berücksichtigen. Die mittels beispielsweise der weiter oben beschriebenen Schätzmethoden für die geschätzte Clearance kann auf Grund der kardiopulmonaren Rezirkulation zu hoch ausfallen. Zur Bestimmung der Rezirkulation im Gefäßzugang kann daher die kardiopulmonare Rezirkulation geschätzt werden, beispielsweise indem ein üblicher Wert oder ein früher bei dem Patienten ermittelter Wert herangezogen wird und damit die geschätzte Clearance reduziert wird. Beispielsweise kann in einer ersten Näherung angenommen werden, dass sich die Gesamtrezirkulation als Summe aus der kardiopulmonaren Rezirkulation und der Rezirkulation im Gefäßzugang darstellt und dementsprechend die geschätzte Clearance um den Anteil der kardiopulmonaren Rezirkulation reduziert wird. In einer anderen Näherung kann aus der geschätzten Clearance und der gemessenen Clearance die Gesamtrezirkulation abgeschätzt werden und dann zur Bestimmung der Rezirkulation im Gefäßzugang die kardiopulmonare Rezirkulation von der Gesamtrezirkulation abgezogen werden;

4b1. Bestimmung des Blutflusswert im Gefäßzugang beispielsweise nach der Formel Qa = (1/R-1)*Qb;

4a2. Bestimmung des Blutflusswert im Gefäßzugang mittels Gleichung (4), und optional eine Bestimmung der Rezirkulation nach der Formel Qa = (1/R-1)*Qb aufgelöst nach R.

Die Genauigkeit der Wege über 4a1 bzw. 4a2 hängt dabei von der Genauigkeit der Schätzungen ab. Die geschätzte Clearance kann aus den Herstellerangaben und den Flüssen berechnet werden. Dann wird die geschätzte Clearance überschätzt, da die Verminderung durch die kardiopulmonare Rezirkulation nicht berücksichtigt wird. Überschätzen der geschätzte Clearance führt beim Weg über die Rezirkulation (Schritt 4a.1) zu hohen Werten für die Rezirkulation und damit zu geringen Werten von Qa (Schritt 4b.1). Direktes Einsetzen der geschätzten Clearance in die Gleichung (4) geht in die gleiche Richtung (Schritt 4a2.). Man kann daher beim Abschätzen der geschätzten Clearance aus den Herstellerangaben einen typischen Wert der kardiopulmonaren Rezirkulation (z.B. 5%, 10%, oder 15%) verwenden, um die geschätzte Clearance besser abzuschätzen.

Zur Bestimmung des Blutflusses im Gefäßzugang kann die Einrichtung zur Erzeugung einer Veränderung einer physikalischen oder chemischen Kenngröße derart ausgebildet sein, dass die Stoffkonzentration eines Stoffes in der Dialysierflüssigkeit, insbesondere die Na-Konzentration, für die Bestimmung des ersten und zweiten Wertes der Clearance unter der ersten und zweiten Dialysebedingung erhöht wird oder für die Bestimmung des ersten und zweiten Wertes der Clearance unter der ersten und zweiten Dialysebedingung verringert wird. Wenn von einer Änderung "eines Stoffes, insbesondere die Na-Konzentration" die Rede ist, bedeutet dies stets auch, dass bei Ionen auch entsprechende Gegenionenkonzentrationen geändert werden müssen. Tatsächlich liegt beispielsweise kein elementares Natrium in der Lösung vor, sondern positiv geladene Natrium-Ionen. Die entsprechenden negativen Gegenionen können Chlorid-Ionen sein. In der Regel sind die negativen Gegenionen Chlorid-Ionen. Andere Gegenionen in einer herkömmlichen Dialysierflüssigkeit sind Carbonat- oder Bicarbonat-Ionen. Mit anderen Worten umfasst die Änderung der Konzentration eines Stoffes die Änderung der Konzentration eines oder mehrere Arten ungeladenener Moleküle, und die Änderung der Konzentration eines Ions und eines Gegenions, die Änderung der Konzentration mehrere Arten von Ionen und Gegenionen.

Der Erfinder hat erkannt, dass ein für die Bestimmung der Dialysedosis Kt/V in der Größe ausreichender Bolus für die Bestimmung des Blutflusses im Gefäßzugang Qa mit einer hinreichenden Genauigkeit und Zuverlässigkeit nicht ausreichend ist. Der Grund hierfür scheint darin zu liegen, dass die Bestimmung des Blutflusses im Gefäßzugang Qa zwei Werte erfordert, wobei in die Gleichung zur Berechnung von Qa zwei Werte eingehen, so dass sich Messfehler viel stärker auswirken können als wenn ein Hämodialyseparameter nur auf der Grundlage einer Messung bestimmt wird.

Eine Ausführungsform der erfindungsgemäßen Dialysevorrichtung sieht eine mit der Einrichtung zur Erzeugung einer Veränderung einer physikalischen oder chemischen Kenngröße und der Einrichtung zur Messung einer zeitlichen Veränderung einer physikalischen oder chemischen Kenngröße zusammenwirkende Steuer- und Recheneinheit vor, die während der Blutbehandlung zur Bestimmung eines Hämodialyseparameters die Erzeugung und Erfassung der Veränderung der physikalischen oder chemischen Kenngröße in Gang setzt. Die Steuer- und Recheneinheit kann die Veränderung und Erfassung der Kenngröße während der Blutbehandlung unter Berücksichtigung bestimmter zeitlicher Vorgaben automatisch vornehmen, wobei der Zeitpunkt für eine einmalige oder zweimalige Bestimmung eines Dialyseparameters oder die Zeitpunkte für eine mehrfache Bestimmung eines Dialyseparameters in einem Speicher der Steuer- und Recheneinheit gespeichert sein können. Der Zeitpunkt bzw. die Zeitpunkte können aber auch über eine Eingabeeinheit vom medizinischen Personal eingegeben und in den Speicher eingelesen werden. Die Steuer- und Recheneinheit kann auch die erforderlichen Auswertungen der Messignale und die Berechnungen durchführen bzw. dazu programmiert sein.

Die Steuer- und Recheneinheit kann derart ausgebildet sein, dass zur Bestimmung des ersten Hämodialyseparameters, insbesondere des Blutflusses im Gefäßzugang Qa, die Erzeugung und Erfassung der Veränderung der physikalischen oder chemischen Kenngröße während der Blutbehandlung nur einmal oder nur zweimal in Gang gesetzt wird. Eine einmalige Bestimmung von Qa ist im Allgemeinen ausreichend. Es hat sich gezeigt, dass für die Bestimmung von Qa mit dem größeren Bolus eine hohe Messgenauigkeit erzielt werden kann. Für die Messung von Qa ist in der Praxis eine Streuung von ca. 20% tolerabel, um eine Trendanalyse bei typischen Blutflüssen von ca. 1200 ml/min zu ermöglichen, was eine Reproduzierbarkeit der Clearance-Messung von 2% unabhängig von der Nadelorientierung voraussetzen kann. Da die Messung von Qa nur einmal pro Dialysebehandlung erfolgt, kann ein größer Bolus eher toleriert werden.

Die Steuer- und Recheneinheit kann derart ausgebildet ist, dass zur Bestimmung des zweiten Hämodialyseparameters die Erzeugung und Erfassung der Veränderung der physikalischen oder chemischen Kenngröße während der Blutbehandlung mehrfach in Gang gesetzt wird, wobei zeitliche Vorgaben automatisch erfolgen können oder vom medizinischen Personal gemacht werden können. Es hat sich gezeigt, dass für die Bestimmung der Dialysedosis Kt/V mit einem Bolus der kleiner ist als der Bolus für die Bestimmung des Blutflusses im Gefäßzugang Qb eine ausreichende Messgenauigkeit erzielt werden kann. Da ein kleiner Bolus für den Patienten keine Belastung darstellen kann, können auch mehrere Messungen während der Blutbehandlung ohne Probleme vorgenommen werden.

Die Einrichtung zur Erzeugung einer Veränderung einer physikalischen oder chemischen Kenngröße kann für die Bestimmung des ersten Hämodialyseparametes derart ausgebildet sein, dass die Stoffkonzentration eines Stoffes in der Dialysierflüssigkeit, insbesondere die Na-Konzentration, für die Bestimmung des ersten und zweiten Wertes der Clearance unter der ersten und zweiten Dialysebedingung erhöht wird oder für die Bestimmung des ersten und zweiten Wertes der Clearance unter der ersten und zweiten Dialysebedingung verringert wird. Die Bestimmung des ersten Hämodialyseparamters mit zwei "negativen" Boli hat den Vorteil, dass die Stoffkonzentration in der Dialysierflüssigkeit, insbesondere der Na-Konzentration, nicht erhöht wird. Eine Erhöhung der Na-Konzentration kann zu einer Erhöhung des Blutdrucks des Patienten führen. Die Bestimmung des ersten Hämodialyseparameters mit zwei "positiven" Boli ist mit einem geringeren Risiko von intradialytischen Blutdruckabfällen und Krämpfen verbunden, die durch eine Verringerung der Na-Konzentration hervorgerufen werden können.

Wenn mehrere Messung zur Bestimmung des zweiten Dialyseparameters durchgeführt werden sollen, kann die Einrichtung zur Erzeugung einer Veränderung einer physikalischen oder chemischen Kenngröße derart ausgebildet sein, dass die Stoffkonzentration eines Stoffes in der Dialysierflüssigkeit, insbesondere die Na-Konzentration, für eine jeweils vorausgehende Messung erhöht und eine nachfolgende Messung verringert wird oder für eine vorausgehende Messung verringert und eine nachfolgende Messung erhöht wird. Die wechselweise Verabreichung eines "positiven" und eines "negativen" Bolus hat den Vorteil einer geringeren Änderung der Stoffkonzentration in der Dialysierflüssigkeit, insbesondere der Na-Konzentration.

Darüber hinaus betrifft die Erfindung ein Computerprogrammprodukt, das Befehle umfasst, die bei der Ausführung des Programms durch einen Computer diesen veranlassen, die oben beschriebenen Verfahren zur Bestimmung von mindestens einem ersten und zweiten Hämodialyseparameter während einer Dialysebehandlung mit einer Dialysevorrichtung auszuführen.

Im Folgenden wird ein Ausführungsbeispiel der Erfindung unter Bezugnahme auf die Figuren im Einzelnen beschrieben.

Es zeigen:

Fig. 1 ein Ausführungsbeispiel der erfindungsgemäßen Dialysevorrichtung in vereinfachter schematischer Darstellung,

Fig. 2A die Einrichtung zur Vorgabe einer Dialysebedingung der Dialysevorrichtung, wobei eine erste Dialysebedingung vorgegeben wird,

Fig. 2B die Einrichtung zur Vorgabe einer Dialysebedingung der Dialysevorrichtung, wobei eine zweite Dialysebedingung vorgegeben wird,

Fig. 3 die zeitliche Veränderung der physikalischen oder chemischen Kenngröße der Dialysierflüssigkeit am Eingang und Ausgang des Dialysators,

Fig. 4 die erste und zweite zeitliche Veränderung der physikalischen oder chemischen Kenngröße der Dialysierflüssigkeit am Eingang des Dialysators für die Bestimmung eines ersten Hämodialyseparameters, wobei ein positiver Bolus verabreicht wird,

Fig. 5 die zeitliche Veränderung der physikalischen oder chemischen Kenngröße der Dialysierflüssigkeit am Eingang des Dialysators für die Bestimmung eines zweiten Hämodialyseparameters,

Fig. 6 die erste und zweite zeitliche Veränderung der physikalischen oder chemischen Kenngröße der Dialysierflüssigkeit am Eingang des Dialysators für die Bestimmung eines ersten Hämodialyseparameters, wobei ein negativer Bolus verabreicht wird,

Fig. 7 eine Folge von positiven und negativen Boli für die fortlaufende Bestimmung des zweiten Hämodialyseparameters während der Blutbehandlung,

Fig. 8 die in einem Versuch gemessene Leitfähigkeit der Dialysierflüssigkeit stromauf und stromab des Dialysators.

Fig. 9 ein Ablaufdiagramm zur Veranschaulichung der Bestimmung eines ersten Dialyseparameters auf der Grundlage einer ersten und einer zweiten Messung eines ersten bzw. zweiten Clearance-Wertes bei einer ersten bzw. zweiten Dialysebedingung,

Fig. 10 ein Ablaufdiagramm zur Veranschaulichung der Bestimmung eines ersten Dialyseparameters auf der Grundlage einer Abschätzung eines ersten Clearance-Wertes und einer Messung eines zweiten Clearance-Wertes bei einer ersten bzw. zweiten Dialysebedingung.

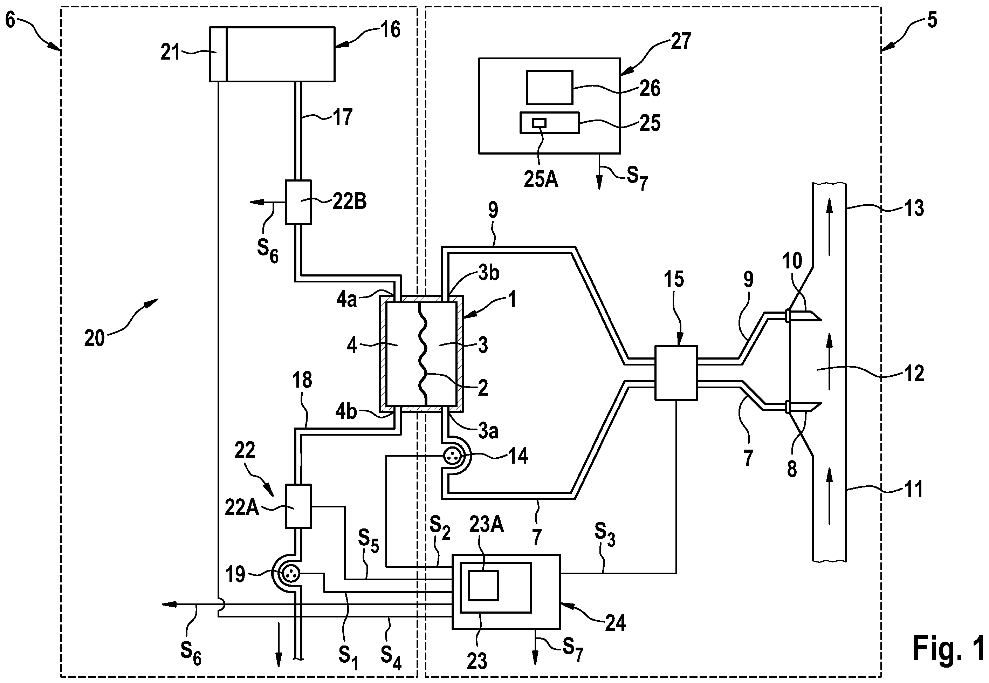

Fig. 1 zeigt die für die Beschreibung der Erfindung wesentlichen Komponenten eines Ausführungsbeispiels der erfindungsgemäßen Dialysevorrichtung in vereinfachter schematischer Darstellung. Da die Messverfahren zur Bestimmung der Hämodialyseparameter als solche zum Stand der Technik gehören, werden nur die für die Erfindung wesentlichen Gesichtspunkte der Verfahren beschrieben.

Im Betrieb ist die Dialysevorrichtung mit einem Dialysator und einem extrakorporalen Blutschlauchsystem verbunden. Die Dialysevorrichtung ist so programmiert, dass sie das erfindungsgemäße Verfahren zur Bestimmung der Dialyseparameter durchführen kann, wenn sie sowohl mit einem Dialysator und einem extrakorporalen Blutschlauchsystem als auch mit einem Patienten verbunden ist.

Die erfindungsgemäße Dialysevorrichtung kann mit einem Dialysator 1 bestückt werden, der durch eine semipermeable Membran 2 in ein erstes Kompartiment 3 und ein zweites Kompartiment 4 unterteilt ist. Das erste Kompartiment 3 des Dialysators 1 ist Teil eines in Fig. 1 in gestrichelten Linien dargestellten extrakorporalen Blutkreislaufs 5, und das zweite Kompartiment 4 des Dialysators 1 ist Teil eines in gestrichelten Linien dargestellten Dialysierflüssigkeitssystems 6.

Der extrakorporale Blutkreislauf 5 umfasst eine erste Blutleitung 7, an deren einem Ende eine erste Kanüle 8 angeschlossen ist, und eine zweite Blutleitung 9, an deren einem Ende eine zweite Kanüle 10 angeschlossen ist. Die erste Kanüle 8 wird an einen stromabwärtigen Teil 11 eines Gefäßzugangs 12 des Patienten angeschlossen, und die zweite Kanüle 10 wird an einen stromaufwärtigen Teil 13 des Gefäßzugangs 12 angeschlossen. Die Flussrichtung des Blutes in dem Gefäßzugang ist mit einem Pfeil gekennzeichnet. Das andere Ende der ersten Blutleitung 7 ist mit einem Einlass 3a des ersten Kompartiments 3 und das andere Ende der zweiten Blutleitung 9 ist mit einem Auslass 3b des ersten Kompartiments 3 verbunden, so dass Blut des Patienten von dem stromabwärtigen Teil 11 des Gefäßzugangs in das erste Kompartiment 3 und aus dem ersten Kompartiment 3 in den stromaufwärtigen Teil 13 des Gefäßzugangs 12 strömt. Das Blut wird im extrakorporalen Blutkreislauf 5 mit einer Blutpumpe 14 gefördert.

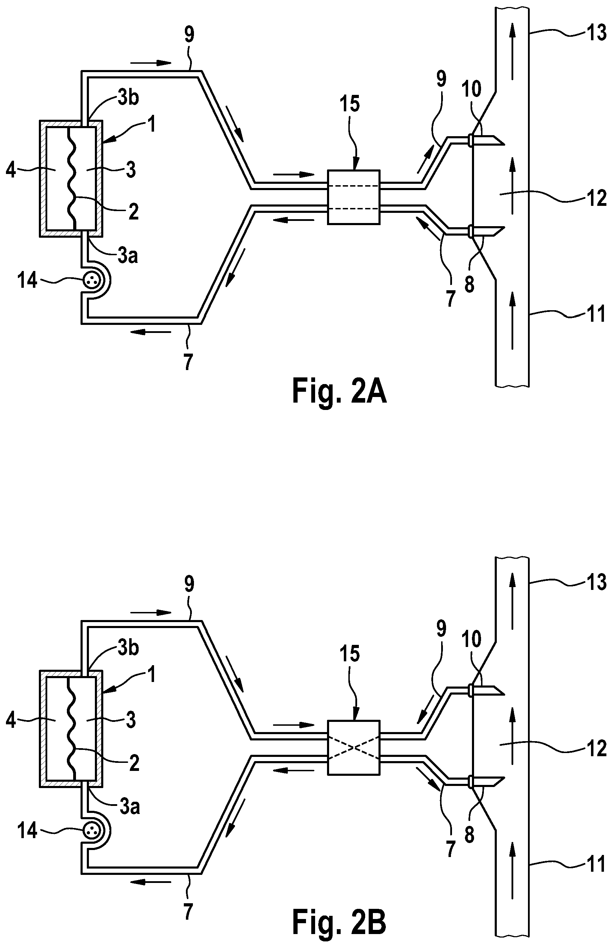

Die Dialysevorrichtung kann eine Einrichtung 15 zur Vorgabe einer ersten und einer zweiten Dialysebedingung aufweisen, die in den Figuren 1A und 1B stark vereinfacht dargestellt ist. Die Einrichtung 15 zur Vorgabe einer Dialysebedingung ist derart ausgebildet, dass eine erste Dialysebedingung vorgegeben werden kann, bei der die erste Blutleitung 7 Blut von dem stromabwärts gelegenen Teil 11 des Gefäßzugangs 12 fördert und die zweite Blutleitung 8 Blut in Richtung auf den stromaufwärts gelegenen Teil 13 des Gefäßzugangs 12 fördert und eine zweite Dialysebedingung vorgegeben werden kann, bei der die erste Blutleitung 7 Blut von dem stromaufwärts gelegenen Teil 13 des Gefäßzugangs 12 fördert und die zweite Blutleitung 9 Blut in Richtung auf den stromabwärts gelegenen Teil 11 des Gefäßzugangs 12 fördert. Fig. 2A zeigt die Richtung des Blutflusses unter der ersten Dialysebedingung und Fig. 2B zeigt die Richtung des Blutflusses unter der zweiten Dialysebedingung. In den gefäßseitigen Leitungsabschnitten ist der Blutfluss umgekehrt.

Die Einrichtung 15 zur Vorgabe einer Dialysebedingung kann eine automatisch betätigbare Anordnung von Ventilen umfassen, die ein Vertauschen von geräteseitigen und patientenseitigen Abschnitten der Blutleitungen erlauben, wie in den Figuren 1A und 1B mit gestrichelten Linien dargestellt ist.

Die Einrichtung 15 kann auch eine manuell zu betätigende Einrichtung sein, die mit der Dialysevorrichtung, insbesondere mit einer Steuereinrichtung der Dialysevorrichtung, nicht verbunden ist. Die Dialysevorrichtung kann über eine Eingabevorrichtung verfügen, mit der das medizinische Personal eingegeben kann, welche Dialysebedingung vorliegen soll. Die Eingabevorrichtung kann auch anzeigen, welche Dialysebedingung vorgegeben wurde bzw. dass die Dialysebedingung verändert wurde, beispielsweise von einer ersten Dialysebedingung zu einer zweiten Dialysebedingung. Eine solche Einrichtung ist beispielsweise der sogenannte Twister® der Firma Fresenius Medical Care Deutschland GmbH.

Die Dialysierflüssigkeit wird mit einer Einrichtung 16 zur Bereitstellung von Dialysierflüssigkeit bereitgestellt, an die eine Dialysierflüssigkeitszuführleitung 17 angeschlossen ist, die zu einem Einlass 4a des zweiten Kompartiments 4 des Dialysators 2 führt. An den Auslass 4b des zweiten Kompartiments 4 ist ein Dialysierflüssigkeitsabführleitung 18 angeschlossen, die zu einem nicht dargestellten Ablauf führt. Die Dialysierflüssigkeit wird in dem Dialysierflüssigkeitssystem 6 mit einer Dialysierflüssigkeitspumpe 19 durch das zweite Kompartiment 4 des Dialysators 1 gefördert.

Die Dialysevorrichtung verfügt über eine Vorrichtung 20 zur Bestimmung eines ersten Hämodialyseparameters und eines zweiten Hämodialyseparameters. Bei dem vorliegenden Ausführungsbeispiel ist der erste Hämodialyseparameter die Dialysedosis Kt/V und der zweite Hämodialyseparameter der Blutfluss im Gefäßzugang Qa (blood access flow).

Die Vorrichtung 20 zur Bestimmung der Hämodialyseparameter umfasst eine Einrichtung 21 zur Erzeugung einer zeitlichen Veränderung einer physikalischen oder chemischen Kenngröße der Dialysierflüssigkeit im Dialysierflüssigkeitssystem 6 in der Dialysierflüssigkeitszuführleitung 17 stromauf des zweiten Kompartiments 4 und eine Einrichtung 22 zur Erfassung der auf die zeitliche Veränderung der Kenngröße stromauf des Dialysators zurückzuführenden zeitlichen Veränderung der Kenngröße in der Dialysierflüssigkeitsabführleitung 18 stromab des zweiten Kompartiments 4. Darüber hinaus kann die Kenngröße auch in der Dialysierflüssigkeitszuführleitung 17 stromauf des zweiten Kompartiments 4 des Dialysators 1 erfasst werden.

Die zeitliche Veränderung der Kenngröße stromauf des Dialysators 1 wird nachfolgend auch als Eingangsbolus und die zeitliche Veränderung der Kenngröße stromab des Dialysators als Ausgangsbolus bezeichnet. Der Ausgangsbolus ist also die Antwort des Systems auf den Eingangsbolus.

Bei dem vorliegenden Ausführungsbeispiel ist die physikalische oder chemische Kenngröße die Konzentration c eines Stoffes in der Dialysierflüssigkeit, insbesondere die Na-Konzentration, die stromauf des Dialysators 1 verändert wird, d. h. für ein vorgegebenes Zeitintervall erhöht oder verringert wird. Die Einrichtung 21 zur Erzeugung einer zeitlichen Veränderung einer physikalischen oder chemischen Kenngröße ist bei dem vorliegenden Ausführungsbeispiel Bestandteil der Einrichtung 16 zur Bereitstellung der Dialysierflüssigkeit, die eine Veränderung der Na-Konzentration des Dialysats für ein vorgegebenes Zeitintervall erlaubt.

Die Einrichtung 22 zur Erfassung der physikalischen oder chemischen Kenngröße weist bei dem vorliegenden Ausführungsbeispiel einen Leitfähigkeitssensor 22A auf, der die Leitfähigkeit der Dialysierflüssigkeit in der Dialysierflüssigkeitsabführleitung 18 als eine mit der Na-Konzentration korrelierende Größe misst. Die Einrichtung 22 zur Erfassung der physikalischen oder chemischen Kenngröße kann einen weiteren Leitfähigkeitssensor 22B aufweisen, der die Leitfähigkeit der Dialysierflüssigkeit in der Dialysierflüssigkeitszuführleitung 17 misst. Wenn die Form des Eingangsbolus bekannt ist, kann der Leitfähigkeitssensor 22B in der Dialysierflüssigkeitszuführleitung 17 entfallen. Ansonsten kann die Leitfähigkeit in der Dialysierflüssigkeitszuführleitung 17 mit dem Leitfähigkeitssensor 22B gemessen werden.

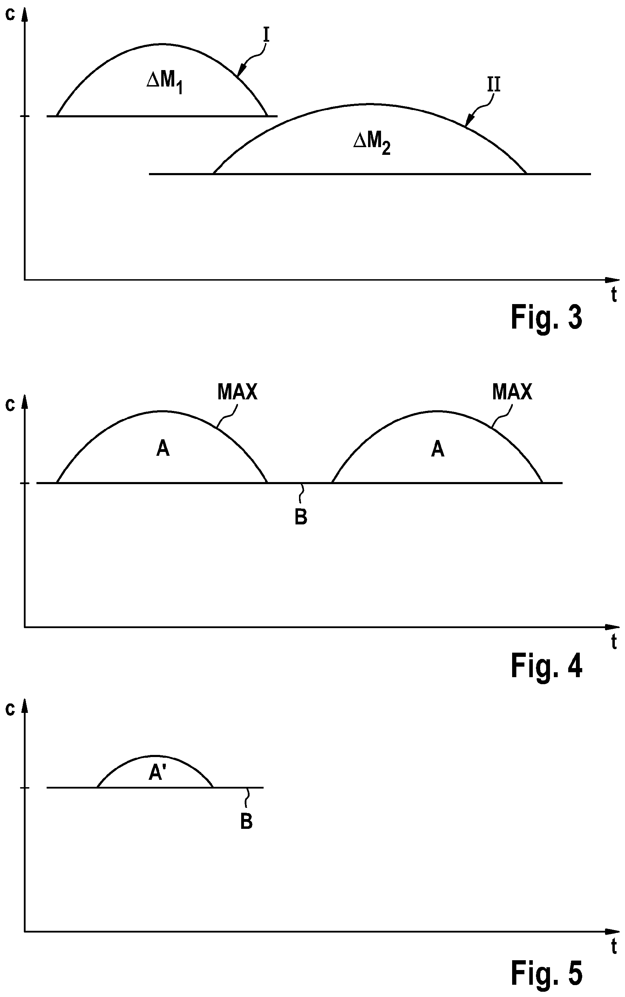

Fig. 3 zeigt den Eingangsbolus und Ausgangsbolus. Der Bolus wird bei dem vorliegenden Ausführungsbeispiel durch den Flächeninhalt der Fläche charakterisiert ist, welche zwischen dem Graphen einer die zeitliche Veränderung der physikalischen oder chemischen Kenngröße beschreibenden Funktion, beispielsweise die Na-Konzentration c(t) bzw. die Leitfähigkeit, und dem Graphen einer Referenzfunktion liegt. Der Flächeninhalt ist ein Maß für die Größe des Bolus, d. h. ein größerer Bolus hat einen größeren Flächeninhalt als ein kleinerer Bolus. Die Referenzfunktion kann eine lineare Funktion sein: f(t)= mt+B sein, wobei m die Steigung der Funktion und B ein vorgegebener Basiswert ist. Die Steigung ist bei dem vorliegenden Ausführungsbeispiel 1. Der Basiswert B kann die Na-Konzentration (Leitfähigkeit) des Blutes vor der Verabreichung des Bolus sein. Der Flächeninhalt des Eingangs- und Ausgangsbolus kann durch Berechnung des Integrals in einem vorgegebenen Integrationsintervall bestimmt werden.

Alternativ oder in Kombination können auch andere Größen zur Charakterisierung des Bolus und somit zur Bestimmung der Hämodialyseparameter herangezogen werden. Eine für den Bolus alternative charakteristische Größe kann die Amplitude und Dauer des Bolus sein. Aus Amplitude und Dauer kann der Flächeninhalt abgeschätzt werden. Auch die Amplitude und Dauer des Bolus allein können aussagekräftige Größen sein, wenn ein Verhältnis zwischen Größe und Dauer eines Bolus bekannt ist.

Im Folgenden wird die Bestimmung der Hämodialyseparameter auf der Grundlage des Flächeninhalts des Bolus beschrieben. Die anderen Größen können in vergleichbarer Weise herangezogen werden.

Die Dialysevorrichtung weist eine mit der Einrichtung 21 zur Erzeugung einer Veränderung einer physikalischen oder chemischen Kenngröße und der Einrichtung 22 zur Erfassung der Kenngröße zusammenwirkende Rechen- und Auswerteeinheit 23 auf, die Bestandteil einer zentralen Rechen- und Steuereinheit 24 der Dialysevorrichtung sein kann. Die zentrale Rechen- und Steuereinheit 24 ist über Steuer- und Signalleitungen $S_1$ bis $S_6$ mit der Dialysierflüssigkeits-pumpe 19, der Blutpumpe 14, der Einrichtung 15 zur Vorgabe der Dialysebedingung, der Einrichtung 21 zur Erzeugung einer Veränderung einer physikalischen oder chemischen Kenngröße (Eingangsbolus) und den Leitfähigkeitssensoren 22A und 22B der Einrichtung 22 zur Erfassung der Kenngröße (Ausgangsbolus) verbunden. Die Rechen- und Steuereinheit 24 ist derart konfiguriert, dass die einzelnen Komponenten der Dialysevorrichtung während der Dialysebehandlung derart angesteuert werden, dass die nachfolgend beschriebenen Verfahrensschritte zur Bestimmung der Hämodialyseparameter durchgeführt werden.

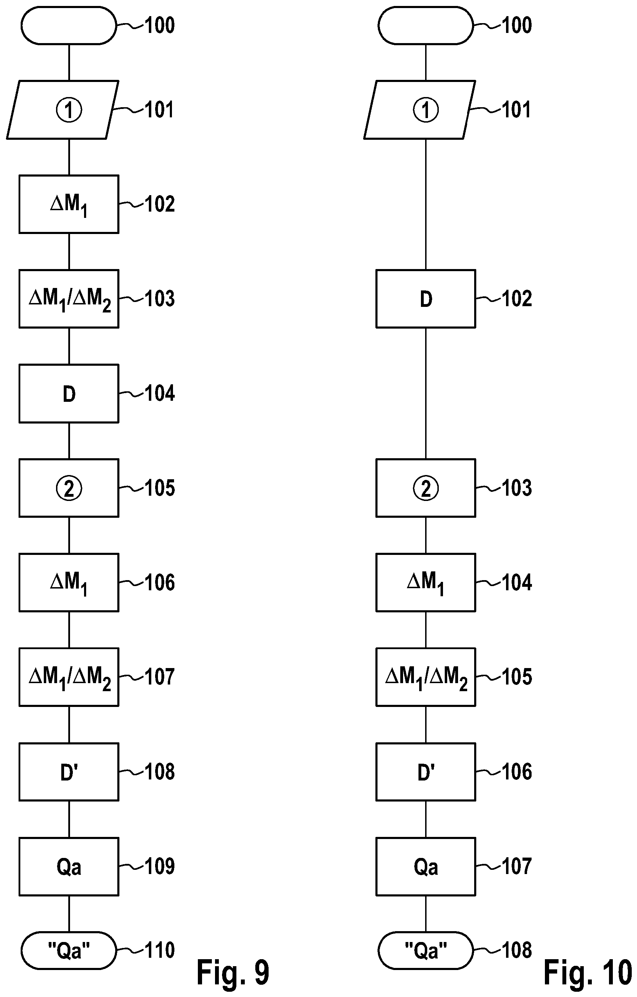

Die einzelnen Verfahrensschritte eines ersten Ausführungsbeispiels zur Bestimmung eines ersten Dialyseparameters sind in Fig. 9 gezeigt.

Die zentrale Steuer- und Recheneinheit 24 kann beispielsweise einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte zur Steuerung der Blutbehandlungsvorrichtung auszuführen. Auf den Hardware-Komponenten kann zur Durchführung der Verfahrensschritte ein Datenverarbeitungsprogramm (Software) laufen.

Darüber hinaus ist eine Eingabeeinheit 25 vorgesehen, die mit einer Signalleitung $S_7$ mit der Rechen- und Steuereinheit 24 verbunden ist. Mit der Eingabeeinheit 25 kann das medizinische Personal die Bestimmung der Hämodialyseparamter betreffende Eingaben machen. Die Eingabeeinheit 25 kann eine Tastatur aufweisen. Zur Anzeige der Hämodialyseparameter ist eine Anzeigeeinheit 26 vorgesehen. Eingabe- und Anzeigeeinheit können auch als ein berührungsempfindlicher Bildschirm 27 (Touchscreen) ausgebildet sein.

Zu Beginn der Dialysebehandlung oder nach Ablauf eines fest vorgegebenen Zeitintervalls oder nach Betätigung eines Button 25A auf dem Touchscreen 27 durch das medizinische Personal wird der Blutfluss im Gefäßzugang Qa (blood acess flow) bestimmt (Schritt 101).

Die Einrichtung 15 zur Vorgabe der Dialysebedingung gibt die erste Dialysebedingung vor (Fig. 2A), um einen ersten Wert für die Clearance (Dialysance) bestimmen zu können (Schritt 102). Die Einrichtung 21 zur Erzeugung einer Veränderung einer physikalischen oder chemischen Kenngröße erzeugt daraufhin einen Eingangsbolus I mit einem vorgegebenen Flächeninhalt $\Delta M_1$ (Schritt 103) (Fig. 3). Die Einrichtung 22 zur Erfassung einer physikalischen oder chemischen Kenngröße erfasst dann den Eingangsbolus I mit dem Flächeninhalt $\Delta M_1$ stromauf des Dialysators 1 und den Ausgangsbolus II mit dem Flächeninhalt $\Delta M_2$ stromab des Dialysators, indem die Leitfähigkeit der Dialysierflüssigkeit mit den Leitfähigkeitssensoren 22A und 22B stromauf- und stromab des Dialysators 1 gemessen wird. Die Messwerte werden in einem Speicher 23A der Rechen- und Auswerteeinheit 24 gespeichert (Schritt 104).

Die Stoffmenge $\Delta M1$, die stromauf des Dialysators zugeführt, und die Stoffmenge $\Delta M2$, die stromab des Dialysators abgeführt wird, ergeben sich wie folgt:

$$\Delta M1 = Qd*\int dcDi*dt \qquad\qquad \text{Gleichung (1)}$$

$$\Delta M2 = Qd*\int dcDo*dt \qquad\qquad \text{Gleichung (2)}$$

wobei Qd der Dialysierflüssigkeitsfluss, cDi die Dialysatkonzentration am Eingang und cDo die Dialysatkonzentration am Ausgang des zweiten Kompartiments 4 des Dialysators 1 und t die Zeit ist.

Die Rechen- und Auswerteeinheit 24 kann einen ersten Wert für die Dialysance D nach der folgenden Gleichung berechnen (Schritt 105):

$$D = Qd*(\Delta Mi - \Delta Mo)/\Delta Mi \qquad\qquad \text{Gleichung (3)}$$

Zur Bestimmung der Dialysance können auch komplexere Gleichungen verwendet werden, die weitere Parameter berücksichtigen, wie beispielsweise Ultrafiltrationsrate (Qf) und/oder Substituatrate (Qs). Diese Gleichungen zur Bestimmung der Dialysance sind schon seit langem bekannt und in der Fachliteratur beschrieben. Eine solche komplexere Gleichung kann beispielsweise folgendermaßen aussehen: $D = (Qd+Qs+Qf)*(1-\Delta M2/\Delta M1)$ und ist von Gleichung (3) als Ausführungsformen umfasst.

Der Dialysierflüssigkeitsfluss Qd wird von der Dialysierflüssigkeitspumpe 19 vorgegeben, die von der Steuer- und Recheneinheit 24 mit der entsprechenden Flussrate angesteuert wird.

Nachdem der erste Wert für die Dialysance bestimmt worden ist, gibt die Einrichtung 21 zur Vorgabe der Dialysebedingung die zweite Dialysebedingung vor (Schritt 106) (Fig. 2B), um einen zweiten Wert für die Clearance (Dialysance) bestimmen zu können. Die Einrichtung 21 zur Erzeugung einer Veränderung einer physikalischen oder chemischen Kenngröße erzeugt daraufhin wieder einen Eingangsbolus mit einem vorgegebenen Flächeninhalt (Schritt 107). Der zweite Eingangsbolus hat vorzugsweise den gleichen oder zumindest annähernd gleichen Flächeninhalt wie der erste Eingangsbolus. Der Leitfähigkeitssensor 22A stromauf des Dialysators 1 erfasst dann den Eingangsbolus und der Leitfähigkeitsmesser 22B stromab des Dialysators den Ausgangsbolus (Schritt 108).

Wie oben bereits ausgeführt, kann die Dialysevorrichtung auch derart programmiert sein, dass die Eingangsboli nicht unmittelbar gemessen, sondern in Kenntnis der zugegeben Natriummenge über die Zeit auch berechnet werden können. Dasselbe gilt bei Kenntnis der Flüsse und des Verhaltens der Dialysierflüssigkeit im Dialysierflüssigkeitssystem für andere die Eingangsboli kennzeichnenden Größen.

Nach der Berechnung von $\Delta M1$ und $\Delta M2$ nach Gleichung (1) bzw. Gleichung (2) berechnet die Rechen- und Auswerteeinheit den zweiten Wert D' für die Dialysance nach Gleichung (3) (Schritt 109).

Den Blutfluss im Gefäßzugang Qa berechnet die Rechen- und Auswerteeinheit nunmehr nach der folgenden Gleichung (Schritt 110):

$$Qa = D*D' / (D - D'), \qquad\qquad \text{Gleichung (4)}$$

wobei D der erste Wert der Dialysance und D' der zweite Wert der Dialysance ist.

Es können auch komplexere Gleichungen verwendet werden, die weitere Parameter berücksichtigen, wie beispielsweise Ultrafiltrationsrate (Qf) und/oder Blutwasserfluß (bwf). Diese Gleichungen zur Bestimmung der Dialysance sind schon seit langem bekannt und in der Fachliteratur beschrieben. Eine solche komplexere Gleichung kann beispielsweise folgendermaßen aussehen: Qa = 1/bwf * (D-Qf)*D'/(D-D') und ist von Gleichung (3) als Ausführungsformen umfasst.

Der Blutfluss im Gefäßzugang Qa wird auf der Anzeigeeinheit 26 angezeigt (Schritt 11).

Fig. 4 zeigt den Eingangsbolus für die erste und zweite Clearance-Messung. Der Flächeninhalt ist in Fig. 4 mit A und der Basiswert ist mit B bezeichnet. Die Einrichtung 21 zur Erzeugung des Eingangsbolus erhöht die Stoffkonzentration der Dialysieflüssigkeit cD, insbesondere die Na-Konzentration, ausgehend von dem Basiswert B auf einen Maximalwert MAX, d. h. die Bestimmung des Blutflusses im Gefäßzugang Qa erfolgt mit zwei positiven Boli, die durch den gleichen Flächeninhalt A charakterisiert sind.

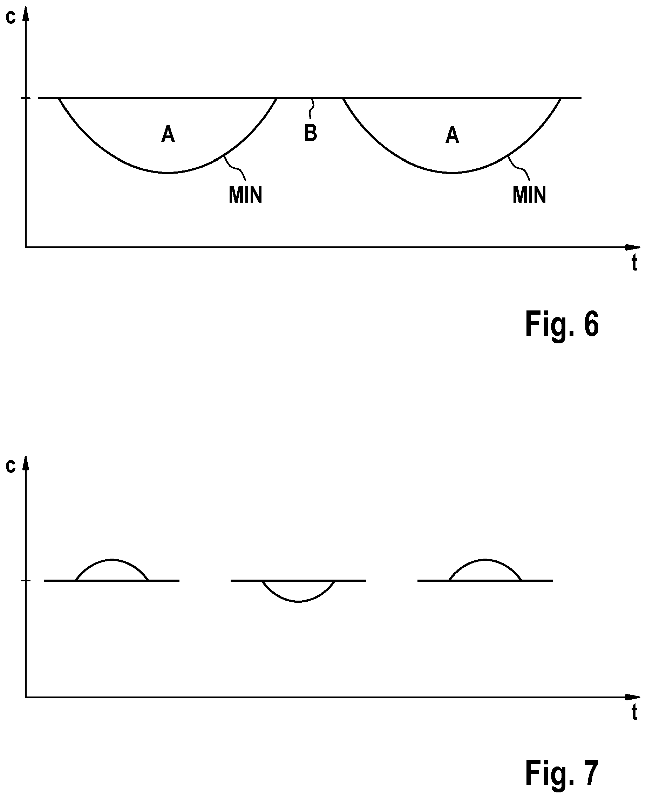

Fig. 6 zeigt eine alternative Ausführungsform mit einem negativen Eingangsbolus für die erste Clearance-Messung und einem negativen Eingangsbolus für die zweite Clearance-Messung. Der Flächeninhalt ist in Fig. 6 wieder mit A und der Basiswert mit B bezeichnet. Die Einrichtung 21 zur Erzeugung des Eingangsbolus verringert die Stoffkonzentration der Dialysieflüssigkeit cD, insbesondere die Na-Konzentration, ausgehend von dem Basiswert B auf einen Minimalwert MIN, d. h. die Bestimmung des Blutflusses im Gefäßzugang Qa erfolgt mit zwei negativen Boli, die durch den gleichen Flächeninhalt A charakterisiert sind.

Vor der Durchführung der Clearance-Messungen für die Bestimmung des ersten Hämodialysparameters kann der Dialysierflüssigkeitsfluss und/oder der Blutfluss auf einen vorgegebenen Wert erhöht werden, indem von der Rechen- und Steuereinheit 24 die Drehzahl der Dialysierflüssigkeitspumpe 19 bzw. der Blutpumpe 14 erhöht wird. Nach der Clearance-Messung kann der Dialysierflüssigkeitsfluss und/oder der Blutfluss wieder auf den zuvor eingestellten Wert verringert werden.

Die Rechen- und Steuereinheit 24 kann nach den Clearance-Messungen für die Bestimmung des ersten Hämodialysparameters auch einen Betriebsmodus vorgeben, der eine Reduktion der Konzentration des verabreichten Stoffes, insbesondere des eingetragenen Salzes, bewirkt. Maßnahmen zur Reduzierung des Salzgehaltes im Dialysat über eine vorgegebene Zeitdauer oder mit einem vorgegebenen Profil sind beispielsweise in der DE 3 223 051 A1 beschrieben.

Das Verfahren zur Clearance-Messung als solches ist in der EP 0 911 043 B1 und das Verfahren der Bestimmung des Blutflusses Qa (blood access flow) im Gefäßzugang auf der Grundlage von zwei aufeinanderfolgenden Clearance-Messungen als solches ist in der EP 0 928 614 B1 im Einzelnen beschrieben.

Die oben beschriebene Bestimmung des Blutflusses im Gefäßzugang erfolgt nur einmal während der Dialysebehandlung automatisch oder nach Betätigung des Button 25A durch das medizinische Personal.

Die erfindungsgemäße Dialysevorrichtung sieht aber nicht nur die Bestimmung des Blutflusses im Gefäßzugang, sondern auch die Bestimmung der Dialysedosis kt/V während der Dialysebehandlung vor.

Während der Dialysebehandlung erzeugt die Einrichtung 21 zur Erzeugung einer physikalischen oder chemischen Eigenschaft vorzugsweise unter der ersten Dialysebedingung einen Eingangsbolus, indem die Na-Konzentration der Dialysierflüssigkeit ausgehend von dem Basiswert B erhöht oder verringert wird.

Fig. 5 zeigt einen Eingangsbolus A', wobei die Na-Konzentration erhöht wird. Die Einrichtung zur Erfassung 22 einer physikalischen oder chemischen Kenngröße erfasst dann den Eingangsbolus stromauf des Dialysators 1 und den Ausgangsbolus stromab des Dialysators, indem die Leitfähigkeit der Dialysierflüssigkeit mit den Leitfähigkeitssensoren 22A, 22B stromauf und stromab des Dialysators 11 gemessen wird. Die Messwerte werden in dem Speicher 23A der Rechen- und Auswerteeinheit 23 gespeichert und die Dialysance D (Clearance K) nach Gleichung (3) berechnet. Nach der Bestimmung der Dialysance D (Clearance K) berechnet die Rechen- und Auswerteeinheit 23 die Dialysedosis Kt/V, die mit der Anzeigeeinheit 26 angezeigt wird.

Während der erste Hämodialyseparameter (blood access flow) nur auf der Grundlage eines Eingangsbolus bestimmt wird, der den Flächeninhalt A hat, wird der zweite Hämodialyseparameter (Dialysedosis) nur auf der Grundlage eines Eingangsbolus bestimmt, der den Flächeninhalt A' hat, wobei der Flächeninhalt A mindestens 2,5 mal so groß, insbesondere mindestens 2,7 mal, insbesondere mindestens 3 mal so groß wie der Flächeninhalt A' ist (Fig. 4, Fig. 5).

Der Quotient zwischen dem Flächeninhalt A und A' des Eingangsbolus für die Bestimmung des ersten und zweiten Hämodialyseparameters kann in Abhängigkeit von dem Signal-Rausch-Verhältnis (signal-to-noise ratio) festgelegt werden. Der Quotient kann in Abhängigkeit von dem Signal-Rausch-Verhältnis mit einem Algorithmus berechnet werden oder die entsprechenden Werte können in dem Speicher 23A der Rechen- und Auswerteeinheit 23 gespeichert werden. In dem Speicher 23A können auch Werte gespeichert werden, die für den Eingangsbolus eine bestimmte Pulsform und/oder Pulsdauer vorgeben.

Während die Bestimmung des Blutflusses im Gefäßzugang Qa mit einem relativ großen Bolus erfolgt, wird die Dialysedosis Kt/V mit einem relativ kleinen Bolus bestimmt. Aufgrund des großen Bolus kann der Blutfluss im Gefäßzugang mit einer hohen Genauigkeit bestimmt werden. Im Allgemeinen ist eine einmalige Bestimmung des Blutflusses im Gefäßzugang vorzugsweise zu Beginn der Dialysebehandlung ausreichend. Die Dialysedosis wird hingegen während der Dialysebehandlung vorzugsweise mehrmals bestimmt.

Fig. 7 zeigt eine Folge von Eingangsboli für die Bestimmung der Dialysedosis. Einem positiven Bolus für eine vorausgehende Messung folgt jeweils ein negativer Bolus für eine nachfolgende Messung, wobei ein positiver Na-Bolus mit einer Erhöhung der Na-Konzentration verbunden ist ein negativer Na-Bolus mit einer Verringerung der Na-Konzentration verbunden ist.

Andere Sequenzen können alternativ oder zusätzlich programmiert sein. Bei einer wechselnden Abfolge zwischen positiven und negativen Boli kann jeder zweite Bolus nach oben gerichtet sein, bei einer Abfolge von positiven Boli, können alle Boli zu größeren Werten als der Basislinie gerichtet sein. Vorzugweise wird das Dialysegerät gemäß wechselnder Boli oder allein mit positiven Boli gesteuert. Die Figuren 3 bis 7 sollen nur der Veranschaulichung dienen.

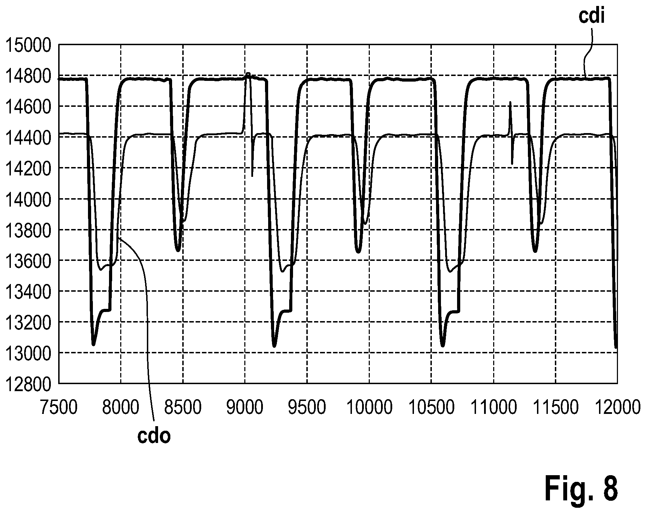

Fig. 8 zeigt die in einem Versuch gemessene Leitfähigkeit stromauf und stromab des Dialysators als Funktion der Zeit t. Die gemessene Leitfähigkeit korreliert mit der Konzentration des Stoffes (Na-Konzentration) in der Dialysierflüssigkeit. In Fig. 8 ist Stoffkonzentration (Leitfähigkeit) stromauf des Dialysators 1 (Eingangsbolus) mit cdi und die Stoffkonzentration (Leitfähigkeit) stromab des Dialysators (Ausgangsbolus) mit cdo bezeichnet.

Für die Leitfähigkeit können Ober- und Untergrenzen vorgegeben werden, die bei der Erzeugung eines positiven bzw. negativen Bolus nicht überschritten bzw. unterschritten werden. Die Grenzen können so vorgegeben werden, dass eine Belastung für den Patienten ausgeschlossen ist.

Bei dem oben beschriebenen Ausführungsbeispiel wird der erste Clearance-Wert auf der Grundlage der Veränderung einer physikalischen oder chemischen Eigenschaft der Dialysierflüssigkeit bestimmt. Eine alternative Ausführungsform zur Bestimmung des Blutflusses im Gefäßzugang Qa sieht anstelle der Messung des ersten Clearance-Wertes D dessen Schätzung vor. Fig. 10 zeigt die einzelnen Verfahrensschritte des alternativen Ausführungsbeispiels. Die Verfahrensschritte 100 und 101 und 103 bis 108 entsprechen den Verfahrensschritten 100, 101 und 105 bis 110 des ersten Ausführungsbeispiels (Fig. 9). Diesbezüglich wird auf die Beschreibung des ersten Ausführungsbeispiels zur Bestimmung des ersten Clearance-Wertes verwiesen. Anstelle der Verfahrensschritte 102, 103 und 104 des ersten Ausführungsbeispiels wird der erste Clearance-Wert D bei dem zweiten Ausführungsbeispiel geschätzt (Schritt 102). Die Rechen- und Auswerteinheit 23 kann einen Schätzwert für den ersten Clearance-Wert nach dem in der EP 1 698 360 B1 beschriebenen Verfahren ermitteln oder nach den Gleichungen berechnen, die in Sargent, A. & Gotch, F., "Principles and biophysics of Dialysis" in Replacement of renewal function by dialysis", 4th ED, veröffentlicht sind.

Ein zweiter Aspekt der Erfindung sieht vor, dass der Abstand der ersten Größe (A) des ersten Bolus zu einer Bezugsgröße größer als der Abstand der zweiten Größe (A') des zweiten Bolus zu einer Bezugsgröße ist. Gemäß dem zweiten Aspekt wird eine Bezugsgröße definiert, wobei die Größe des mindestens einen Bolus für die Bestimmung des ersten Hämodialyseparameters und die Größe des mindestens einen Bolus für die Bestimmung des zweiten Hämodialyseparameters auf die Bezugsgröße bezogen werden. Diese Bezugsgröße ist bei dem vorliegenden Ausführungsbeispiel das Rauschen im System, wobei der Abstand zu der Bezugsgröße der Signal-Rausch-Abstand ist. Für eine genaue Messung wird grundsätzlich ein möglichst großer Signal-Rausch-Abstand angestrebt, der mit zunehmender Größe des Bolus zunimmt, womit aber auch die Belastung für den Patienten zunimmt. Die einzelnen Verfahrensschritte des zweiten Aspekts der Erfindung entsprechen denen des ersten Aspekts, wobei die Dialysevorrichtung für die Bestimmung des ersten Hamödialyseparameters, insbesondere des Blutflusses im Gefäßzugang Qa, einen Bolus vorgibt, der einen größeren Signal-Rausch-Abstand hat als der Bolus für die Bestimmung des zweiten Hamödialyseparameters, insbesondere der Dialysedosis.

## Patentansprüche

1. Dialysevorrichtung eingerichtet zum Verbinden eines Dialysators (1), der durch eine semipermeable Membran (2) in ein erstes Kompartiment (3), das Teil eines extrakorporalen Blutkreislaufs (5) ist, und ein zweites Kompartiment (4), das Teil eines Dialysierflüssigkeitssystems (6) der Dialysevorrichtung ist, unterteilt ist, aufweisend eine Vorrichtung (22) zur Bestimmung von mindestens einem ersten und zweiten Hämodialyseparameter, wobei die Vorrichtung zur Bestimmung eines Hämodialyseparameters aufweist:

   eine Einrichtung (21) zur Erzeugung einer zeitlichen Veränderung einer physikalischen oder chemischen Kenngröße einer Dialysierflüssigkeit in einer Dialysierflüssigkeitszuführleitung (17) zu dem Dialysator (1) im Dialysierflüssigkeitssystem (6) in Form eines Bolus,
   eine Einrichtung (22) zur Erfassung einer auf den Bolus zurückzuführenden zeitlichen Veränderung der physikalischen oder chemischen Kenngröße der Dialysierflüssigkeit in einer Dialysierflüssigkeitsabführleitung (18) aus dem Dialysator im Dialysierflüssigkeitssystem (6),
   eine mit der Einrichtung (21) zur Erzeugung einer zeitlichen Veränderung einer physikalischen oder chemischen Kenngröße und der Einrichtung (22) zur Erfassung einer zeitlichen Veränderung einer physikalischen oder chemischen Kenngröße zusammenwirkenden Rechen- und Auswerteeinheit (23), die derart konfiguriert ist, dass auf der Grundlage einer Veränderung der physikalischen oder chemischen Kenngröße mindestens ein Wert für die Clearance der Dialysebehandlung bestimmt wird, und auf der Grundlage des mindestens einen Wertes für die Clearance mindestens ein Hämodialyseparameter bestimmt wird,

**dadurch gekennzeichnet, dass**

die Einrichtung (21) zur Erzeugung einer Veränderung einer physikalischen oder chemischen Kenngröße derart ausgebildet ist,

dass zur Bestimmung eines ersten Hämodialyseparameters mindestens ein Bolus für die Bestimmung des ersten Hämodialyseparameters mit einer ersten Größe (A) erzeugt wird, wobei die Rechen- und Auswerteeinheit (23) derart konfiguriert ist, dass auf der Grundlage der Veränderung der physikalischen oder chemischen Kenngröße in Form mindestens eines Bolus für die Bestimmung des ersten Hämodialyseparameters mindestens ein Wert für die Clearance der Dialysebehandlung bestimmt wird, auf dessen bzw. auf deren Grundlage der erste Hämodialyseparameter bestimmt wird, und

dass zur Bestimmung eines zweiten Hämodialyseparameters mindestens ein Bolus für die Bestimmung des zweiten Hämodialyseparameters mit einer zweiten Größe (A') erzeugt wird, wobei die Rechen- und Auswerteeinheit (23) derart konfiguriert ist, dass auf der Grundlage der Veränderung der physikalischen oder chemischen Kenngröße in Form mindestens eines Bolus für die Bestimmung des zweiten Hämodialyseparameters mindestens ein Wert für die Clearance der Dialysebehandlung bestimmt wird, auf dessen bzw. auf deren Grundlage der zweite Hämodialyseparameter bestimmt wird,

wobei die erste Größe (A) größer als die zweite Größe (A') ist oder der Abstand der ersten Größe (A) zu einer Bezugsgröße größer als der Abstand der zweiten Größe (A') zu einer Bezugsgröße ist.

2. Dialysevorrichtung nach Anspruch 1, **dadurch gekennzeichnet, dass** der mindestens eine Bolus für die Bestimmung des ersten Hämodialyseparameters durch eine erste Fläche und der mindestens eine Bolus für die Bestimmung des zweiten Hämodialyseparameters durch eine zweite Fläche charakterisiert ist, wobei der erste Flächeninhalt der ersten Fläche größer als der zweite Flächeninhalt der zweiten Fläche ist.

3. Dialysevorrichtung nach Anspruch 1 oder 2, **dadurch gekennzeichnet, dass** der erste Hämodialyseparameter ein Blutfluss im Gefäßzugang (Qa) oder ein Rezirkulationsfluss in einem Gefäßsystem ist.

4. Dialysevorrichtung nach Anspruch 3, **dadurch gekennzeichnet, dass** die Dialysevorrichtung eingerichtet ist zum Verbinden mit einem extrakorporalen Blutkreislauf (5), der eine erste Blutleitung (7) und eine zweite Blutleitung (9) umfasst, die mit einem Eingang (3a) bzw. Ausgang (3b) des ersten Kompartiments (3) des Dialysators (1) zu verbinden ist, und

dass zur Bestimmung des Blutflusses im Gefäßzugang (Qa) die Einrichtung (21) zur Erzeugung einer Veränderung einer physikalischen oder chemischen Kenngröße derart ausgebildet ist, dass für eine erste Dialysebedingung ein erster Bolus mit dem ersten Flächeninhalt (A) und für eine zweite Dialysebedingung ein zweiter Bolus mit dem ersten Flächeninhalt (A) erzeugt wird, wobei die Rechen- und Auswerteeinheit (24), derart konfiguriert ist, dass auf der Grundlage der Veränderung der physikalischen oder chemischen Kenngröße in Form des ersten Bolus mit dem ersten Flächeninhalt (A) unter der ersten Dialysebedingung ein erster Wert für die Clearance und auf der Grundlage der Veränderung der physikalischen oder chemischen Kenngröße in Form des zweiten Bolus unter der zweiten Dialysebedingung ein zweiter Wert für die Clearance bestimmt wird, auf deren Grundlage der Blutfluss im Gefäßzugang bestimmt wird,

**oder**

zur Bestimmung des Blutflusses im Gefäßzugang (Qa) die Einrichtung (21) zur Erzeugung einer Veränderung einer physikalischen oder chemischen Kenngröße derart ausgebildet ist, dass für eine zweite Dialysebedingung ein Bolus mit dem ersten Flächeninhalt (A) erzeugt wird, wobei die Rechen- und Auswerteeinheit, derart konfiguriert ist, dass ein erster Wert für die Clearance auf der Grundlage einer Schätzung der Clearance für die erste Dialysebedingung und auf der Grundlage der Veränderung der physikalischen oder chemischen Kenngröße in Form des Bolus mit dem ersten Flächeninhalt (A) unter der zweiten Dialysebedingung ein zweiter Wert für die Clearance bestimmt wird, auf deren Grundlage der Blutfluss im Gefäßzugang bestimmt wird, wobei

unter der ersten Dialysebedingung die erste Blutleitung (7) Blut von einem stromabwärts gelegenen Teil (11) eines Gefäßzugangs (12) des Patienten fördert und die zweite Blutleitung (9) Blut in Richtung auf einen stromaufwärts gelegenen Teil (13) des Gefäßzugangs (11) fördert und unter der zweiten Dialysebedingung die erste Blutleitung (7) Blut von einem stromaufwärts gelegenen Teil (13) des Gefäßzugangs (12) fördert und die zweite Blutleitung (9) Blut in Richtung auf einen stromabwärts gelegenen Teil (11) des Gefäßzugangs (11) fördert.

5. Dialysevorrichtung nach einem der Ansprüche 1 bis 4, **dadurch gekennzeichnet, dass** der zweite Hämodialyseparameter die Dialysedosis Kt/V ist, wobei K die Clearance, t die Dialysedauer und V das Harnstoff-Verteilungsvolumen ist.

6. Dialysevorrichtung nach Anspruch 5, **dadurch gekennzeichnet, dass** zur Bestimmung der Dialysedosis Kt/V die Einrichtung (21) zur Erzeugung einer Veränderung einer physikalischen oder chemischen Kenngröße derart ausgebildet ist, dass ein Bolus mit dem zweiten Flächeninhalt (A') erzeugt wird, wobei die Rechen- und Auswerteeinheit (23), derart konfiguriert ist, dass auf der Grundlage der Veränderung der physikalischen oder chemischen Kenngröße in Form des Bolus mit dem zweiten Flächeninhalt (A') ein Wert für die Clearance bestimmt wird, auf dessen Grundlage die Dialysedosis Kt/V bestimmt wird.

7. Dialysevorrichtung nach einem der Ansprüche 1 bis 6, **dadurch gekennzeichnet, dass** eine mit der Einrichtung (21) zur Erzeugung einer Veränderung einer physikalischen oder chemischen Kenngröße und der Einrichtung (22) zur Erfassung einer zeitlichen Veränderung einer physikalischen oder chemischen Kenngröße zusammenwirkende Steuer- und Recheneinheit (24) vorgesehen ist, die derart konfiguriert ist, dass während der Blutbehandlung zur Bestimmung des ersten oder zweiten Hämodialyseparameters die Erzeugung und Erfassung der Veränderung der physikalischen oder chemischen Kenngröße in Gang gesetzt wird.

8. Dialysevorrichtung nach Anspruch 7, **dadurch gekennzeichnet, dass** die Steuer- und Recheneinheit (24) derart konfiguriert ist,

dass zur Bestimmung des ersten Hämodialyseparameters die Erzeugung und Erfassung der Veränderung der physikalischen oder chemischen Kenngröße während der Blutbehandlung nur einmal oder nur zweimal in Gang gesetzt wird,
und/oder
dass zur Bestimmung des zweiten Hämodialyseparameters die Erzeugung und Erfassung der Veränderung der physikalischen oder chemischen Kenngröße während der Blutbehandlung mehrfach in Gang gesetzt wird.

9. Dialysevorrichtung nach einem der Ansprüche 4 bis 8, **dadurch gekennzeichnet, dass** zur Bestimmung des ersten Hämodialyseparameters die Einrichtung (21) zur Erzeugung einer Veränderung einer physikalischen oder chemischen Kenngröße derart ausgebildet ist, dass die Stoffkonzentration eines Stoffes in der Dialysierflüssigkeit, insbesondere die Na-Konzentration, für die Bestimmung des ersten und zweiten Wertes der Clearance unter der ersten und zweiten Dialysebedingung bzw. des Wertes der Clearance unter der zweiten Dialysebedingung erhöht wird oder für die Bestimmung des ersten und zweiten Wertes der Clearance unter der ersten und zweiten Dialysebedingung bzw. des Wertes der Clearance unter der zweiten Dialysebedingung verringert wird,
und/oder
zur Bestimmung des zweiten Hämodialyseparameters die Einrichtung (21) zur Erzeugung einer Veränderung einer physikalischen oder chemischen Kenngröße derart ausgebildet ist, dass die Stoffkonzentration eines Stoffes in der Dialysierflüssigkeit, insbesondere die Na-Konzentration, für eine vorausgehende Messung erhöht und eine nachfolgende Messung verringert wird oder für eine vorausgehende Messung verringert und eine nachfolgende Messung erhöht wird.

10. Dialysesystem mit einer Dialysevorrichtung nach einem der Ansprüche 1 bis 9 und mit einem Dialysator, der der durch eine semipermeable Membran (2) in ein erstes Kompartiment (3), das Teil eines extrakorporalen Blutkreislaufs (5) ist, und ein zweites Kompartiment (4), das Teil eines Dialysierflüssigkeitssystems (6) der Dialysevorrichtung ist, unterteilt ist, und mit einem extrakorporalen Blutkreislauf, der eine erste Blutleitung (7) und eine zweite Blutleitung (9) umfasst, die mit einem Eingang (3a) bzw. Ausgang (3b) des ersten Kompartiments (3) des Dialysators (1) verbunden ist.

11. Computerprogrammprodukt, das Befehle umfasst, die bei der Ausführung des Programms durch einen Computer diesen veranlassen, ein Verfahren zur Bestimmung von mindestens einem ersten und zweiten Hämodialyseparameter während einer Dialysebehandlung mit einer Dialysevorrichtung auszuführen, wobei das Verfahren folgende Verfahrensschritte umfasst:

Erzeugen mindestens einer zeitlichen Veränderung einer physikalischen oder chemischen Kenngröße einer Dialysierflüssigkeit in einer Dialysierflüssigkeitszuführleitung (17) zu einem Dialysator (1) in einem Dialysierflüssigkeitssystem (6) in Form eines Bolus,
Erfassen mindestens einer auf den mindestens einen Bolus zurückzuführenden zeitlichen Veränderung der physikalischen oder chemischen Kenngröße der Dialysierflüssigkeit in einer Dialysierflüssigkeitsabführleitung (18) aus dem Dialysator im Dialysierflüssigkeitssystem (6),
Bestimmen mindestens eines Wertes für die Clearance der Dialysebehandlung auf der Grundlage der mindestens einen Veränderung der physikalischen oder chemischen Kenngröße in Form eines Bolus, und Bestimmen mindestens eines Hämodialyseparameters auf der Grundlage des mindestens einen Wertes für die Clearance, wobei

zur Bestimmung eines ersten Hämodialyseparameters mindestens ein Bolus für die Bestimmung des ersten Hämodialyseparameters mit einer ersten Größe (A) erzeugt wird, wobei auf der Grundlage der Veränderung der physikalischen oder chemischen Kenngröße in Form mindestens eines Bolus für die Bestimmung des ersten Hämodialyseparameters mindestens ein Wert für die Clearance der Dialysebehandlung bestimmt wird, auf dessen bzw. auf deren Grundlage der erste Hämodialyseparameter bestimmt wird, und

dass zur Bestimmung eines zweiten Hämodialyseparameters mindestens ein Bolus für die Bestimmung des zweiten Hämodialyseparameters mit einer zweiten Größe (A') erzeugt wird, wobei auf der Grundlage der Veränderung der physikalischen oder chemischen Kenngröße in Form mindestens eines Bolus für die Bestimmung des zweiten Hämodialyseparameters mindestens ein Wert für die Clearance der Dialysebehandlung bestimmt wird, auf dessen bzw. auf deren Grundlage der zweite Hämodialyseparameter bestimmt wird,

wobei die erste Größe (A) größer als die zweite Größe (A') ist oder der Abstand der ersten Größe (A) zu einer Bezugsgröße größer als der Abstand der zweiten Größe (A') zu einer Bezugsgröße ist.

12. Computerprogrammprodukt nach Anspruch 11, wobei das Verfahren **dadurch gekennzeichnet ist, dass** der mindestens eine Bolus für die Bestimmung des ersten Hämodialyseparameters durch eine erste Fläche und der mindestens eine Bolus für die Bestimmung des zweiten Hämodialyseparameters durch eine zweite Fläche charakterisiert sind, wobei der erste Flächeninhalt der ersten Fläche größer als der zweite Flächeninhalt der zweiten Fläche ist.

13. Computerprogrammprodukt nach Anspruch 11 oder 12, wobei das Verfahren **dadurch gekennzeichnet ist, dass** der erste Hämodialyseparameter ein Blutfluss im Gefäßzugang (Qa) oder ein Rezirkulationsfluss in einem Gefäßsystem ist.

14. Computerprogrammprodukt nach Anspruch 13, wobei das Verfahren **dadurch gekennzeichnet ist, dass**

zur Bestimmung des Blutflusses im Gefäßzugang (Qa) für eine erste Dialysebedingung ein erster Bolus mit dem ersten Flächeninhalt (A) und für eine zweite Dialysebedingung ein zweiter Bolus mit dem ersten Flächeninhalt (A) erzeugt wird, wobei auf der Grundlage der Veränderung der physikalischen oder chemischen Kenngröße in Form des ersten Bolus mit dem ersten Flächeninhalt (A) unter der ersten Dialysebedingung ein erster Wert für die Clearance und auf der Grundlage der Veränderung der physikalischen oder chemischen Kenngröße in Form des zweiten Bolus unter der zweiten Dialysebedingung ein zweiter Wert für die Clearance bestimmt wird, auf deren Grundlage der Blutfluss im Gefäßzugang bestimmt wird,
**oder**
zur Bestimmung des Blutflusses im Gefäßzugang (Qa) für eine zweite Dialysebedingung ein Bolus mit dem ersten Flächeninhalt (A) erzeugt wird, wobei ein erster Wert für die Clearance auf der Grundlage einer Schätzung der Clearance für die erste Dialysebedingung und auf der Grundlage der Veränderung der physikalischen oder chemischen Kenngröße in Form des Bolus mit dem ersten Flächeninhalt (A) unter der zweiten Dialysebedingung ein zweiter Wert für die Clearance bestimmt wird, auf deren Grundlage der Blutfluss im Gefäßzugang bestimmt wird,
wobei
unter der ersten Dialysebedingung die erste Blutleitung (7) Blut von einem stromabwärts gelegenen Teil (11) eines Gefäßzugangs (12) des Patienten fördert und die zweite Blutleitung (9) Blut in Richtung auf einen stromaufwärts gelegenen Teil (13) des Gefäßzugangs (11) fördert und unter der zweiten Dialysebedingung die erste Blutleitung (7) Blut von einem stromaufwärts gelegenen Teil (13) des Gefäßzugangs (12) fördert und die zweite Blutleitung (9) Blut in Richtung auf einen stromabwärts gelegenen Teil (11) des Gefäßzugangs (11) fördert.

15. Computerprogrammprodukt nach einem der Ansprüche 12 bis 14, **dadurch gekennzeichnet, dass** der zweite Hämodialyseparameter die Dialysedosis Kt/V ist, wobei K die Clearance, t die Dialysedauer und V das Harnstoff-Verteilungsvolumen ist, und dass zur Bestimmung der Dialysedosis Kt/V ein Bolus mit dem zweiten Flächeninhalt (A') erzeugt wird, wobei auf der Grundlage der Veränderung der physikalischen oder chemischen Kenngröße in Form des Bolus mit dem zweiten Flächeninhalt (A') ein Wert für die Clearance bestimmt wird, auf dessen Grundlage die Dialysedosis Kt/V bestimmt wird.

Claims

1. A dialysis device configured for connecting a dialyser (1) that is divided by a semipermeable membrane (2) into a first compartment (3), which is part of an extracorporeal blood circuit (5), and a second compartment (4), which is part of a dialysis fluid system (6) of the dialysis machine, comprising
a device (22) for determining at least one first and second haemodialysis parameter, the device for determining a haemodialysis parameter comprising:

a device (21) for generating a change over time in a physical or chemical parameter of a dialysis fluid (I) in a dialysis fluid feed line (17) to the dialyser (1) in the dialysis fluid system (6) in the form of a bolus,
a device (22) for detecting a change over time in the physical or chemical characteristic of the dialysis fluid (II) attributable to the bolus in a dialysis liquid discharge line (18) from the dialyser in the dialysis fluid system (6),
a computing and evaluating unit (23) cooperating with the device (21) for generating a change over time in a physical or chemical parameter and the device (22) for detecting a change over time in a physical or chemical parameter that is configured such that, on the basis of a change in the physical or chemical parameter, at least one value for the clearance of the dialysis treatment is determined, and, on the basis of the at least one value for the clearance, at least one haemodialysis parameter is determined,
**characterised in that**
the device (21) for generating a change in a physical or chemical parameter is designed such that,
to determine a first haemodialysis parameter, at least one bolus for determining the first haemodialysis parameter is generated having a first size (A), the computing and evaluating unit (23) being configured such that, on the basis of the change in the physical or chemical parameter in the form of at least one bolus for determining the first haemodialysis parameter, at least one value for the clearance of the dialysis treatment is determined, on the basis of which value or values the first haemodialysis parameter is determined, and
**in that**, to determine a second haemodialysis parameter, at least one bolus for determining the second haemodialysis parameter is generated having a second size (A'), the computing and evaluating unit (23) being configured such that, on the basis of the change in the physical or chemical parameter in the form of at least one bolus for determining the second haemodialysis parameter, at least one value for the clearance of the dialysis treatment is determined, on the basis of which value or values the second haemodialysis parameter is determined,
the first size (A) being larger than the second size (A') or the distance between the first size (A) and a reference value being greater than the distance between the second size (A') and a reference value.

2. The dialysis device according to claim 1, **characterised in that** the at least one bolus for determining the first haemodialysis parameter is **characterised by** a first area, and the at least one bolus for determining the second haemodialysis parameter is **characterised by** a second area, the first area content of the first area being greater than the second area content of the second area.

3. The dialysis device according to claim 1 or claim 2, **characterised in that** the first haemodialysis parameter is a blood flow in the vascular access (Qa) or a recirculation flow in a vascular system.

4. The dialysis device according to claim 3, **characterised in that** the dialysis device is configured for connection to an extracorporeal blood circuit (5) that comprises a first bloodline (7) and a second bloodline (9) that is to be connected to an inlet (3a) or outlet (3b) of the first compartment (3) of the dialyser (1), and

**in that** for determining the blood flow in the vascular access (Qa), the device (21) for generating a change in a physical or chemical parameter is designed such that, for a first dialysis condition, a first bolus having the first area content (A) and, for a second dialysis condition, a second bolus having the first area content (A) is generated, the computing and evaluating unit (24) being configured such that, on the basis of the change in the physical or chemical parameter in the form of the first bolus having the first area content (A) under the first dialysis condition, a first value for the clearance is determined and, on the basis of the change in the physical or chemical parameter in the form of the second bolus under the second dialysis condition, a second value for the clearance is determined, on the basis of which values the blood flow in the vascular access is determined,
**or,**
to determine the blood flow in the vascular access (Qa), the device (21) for generating a change in a physical or chemical parameter is designed such that a bolus having the first area content (A) is generated for a second dialysis condition, the computing and evaluation unit being configured such that, on the basis of an estimate of the clearance for the first dialysis condition, a first value for the clearance is determined and, on the basis of the change in the physical or chemical parameter in the form of the bolus having the first area content (A) under the second dialysis condition, a second value for clearance is determined, on the basis of which values the blood flow in the vascular access is determined,

wherein,

under the first dialysis condition, the first bloodline (7) conveys blood from a downstream portion (11) of a vascular access (12) of the patient and the second bloodline (9) conveys blood towards an upstream portion (13) of the vascular access (11) and, under the second dialysis condition, the first bloodline (7) conveys blood from an upstream part (13) of the vascular access (12) and the second bloodline (9) conveys blood towards a downstream portion (11) of the vascular access (11).

5. The dialysis device according to any of claims 1 to 4, **characterised in that** the second haemodialysis parameter is the dialysis dose Kt/V, K being the clearance, t the dialysis time and V the urea distribution volume.

6. The dialysis device according to claim 5, **characterised in that** for determining the dialysis dose Kt/V, the device (21) for generating a change in a physical or chemical parameter is designed such that a bolus is generated having the second area content (A'), the computing and evaluating unit (23) be configured such that, on the basis of the change in the physical or chemical parameter in the form of the bolus having the second area content (A'), a value for the clearance is determined, on the basis of which value the dialysis dose Kt/V is determined.

7. The dialysis device according to any of claims 1 to 6, **characterised in that** a controlling and computing unit cooperating with the device (21) for generating a change in a physical or chemical parameter and the device (22) for detecting a change over time in a physical or chemical parameter (24) is provided, which controlling and computing unit is configured such that the generation and detection of the change in the physical or chemical parameter is started during the blood treatment to determine the first or second haemodialysis parameter.

8. The dialysis device according to claim 7, **characterised in that** the controlling and computing unit (24) is configured such that, to determine the first haemodialysis parameter, the generation and detection of the change in the physical or chemical parameter is started only once or only twice during the blood treatment

and/or

**in that**, to determine the second haemodialysis parameter, the generation and detection of the change in the physical or chemical parameter is started multiple times during the blood treatment.

9. The dialysis device according to any of claims 4 to 8, **characterised in that**, to determine the first haemodialysis parameter, the device (21) for generating a change in a physical or chemical parameter is designed such that the substance concentration of a substance in the dialysis fluid, in particular the Na concentration, is increased to determine the first and second values of the clearance under the first and second dialysis conditions or the value of the clearance under the second dialysis condition or decreased to determine the first and second values of the clearance under the first and second dialysis conditions or the value of the clearance under the second dialysis condition

and/or

to determine the second haemodialysis parameter, the device (21) for generating a change in a physical or chemical parameter is designed such that the substance concentration of a substance in the dialysis fluid, in particular the Na concentration, is increased for a previous measurement and decreased for a subsequent measurement or decreased for a previous measurement and increased for a subsequent measurement.

10. A dialysis system comprising a dialysis device according to any of claims 1 to 9, and comprising a dialyser that is divided by a semi-permeable membrane (2) into a first compartment (3), which is part of an extracorporeal blood circuit (5), and a second compartment (4), which is part of a dialysis fluid system (6) of the dialysis device, and comprising an extracorporeal blood circuit that comprises a first bloodline (7) and a second bloodline (9) that is connected to an inlet (3a) or outlet (3b) of the first compartment (3) of the dialyser (1).

11. A computer program product that comprises commands that, when the program is executed by a computer, cause the computer to carry out a method for determining at least one first and second haemodialysis parameter during a dialysis treatment with a dialysis device, wherein the method comprises the following method steps:

Generating at least one change over time in a physical or chemical parameter of a dialysis fluid (I) in a dialysis fluid feed line (17) to a dialyser (1) in a dialysis fluid system (6) in the form of a bolus,
Detecting at least one change over time in the physical or chemical parameter of the dialysis fluid (II) attributable to the at least one bolus in a dialysis fluid discharge line (18) from the dialyser in the dialysis fluid system (6),

Determining at least one value for the clearance of the dialysis treatment on the basis of the at least one change in the physical or chemical parameter in the form of a bolus, and determining at least one haemodialysis parameter on the basis of the at least one value for the clearance,

wherein,

to determine a first haemodialysis parameter, at least one bolus for determining the first haemodialysis parameter is generated having a first size (A), at least one value for the clearance of the dialysis treatment being determined on the basis of the change in the physical or chemical parameter in the form of at least one bolus for determining the first haemodialysis parameter, on the basis of which value the first haemodialysis parameter is determined, and

to determine a second haemodialysis parameter, at least one bolus for determining the second haemodialysis parameter is generated having a second size (A'), at least one value for the clearance of the dialysis treatment being determined on the basis of the change in the physical or chemical parameter in the form of at least one bolus for determining the second haemodialysis parameter, on the basis of which value the second haemodialysis parameter is determined,

the first size (A) being larger than the second size (A') or the distance between the first size (A) and a reference value being greater than the distance between the second size (A') and a reference value.

12. The computer program product according to claim 11, wherein the method is **characterised in that** the at least one bolus for determining the first haemodialysis parameter is **characterised by** a first area and the at least one bolus for determining the second haemodialysis parameter is **characterised by** a second area, the first area content of the first area being greater than the second area content of the second area.

13. The computer program product according to claim 11 or claim 12, wherein the method is **characterised in that** the first haemodialysis parameter is a blood flow in the vascular access (Qa) or a recirculation flow in a vascular system.

14. The computer program product according to claim 13, wherein the method is **characterised in that**,

to determine the blood flow in the vascular access (Qa), a first bolus having the first area content (A) is generated for a first dialysis condition and a second bolus having the first area content (A) is generated for a second dialysis condition, a first value for the clearance being determined on the basis of the change in the physical or chemical parameter in the form of the first bolus having the first area content (A) under the first dialysis condition and a second value for the clearance being determined on the basis of the change in the physical or chemical parameter in the form of the second bolus under the second dialysis condition, on the basis of which values the blood flow in the vascular access is determined,

**or**

, to determine the blood flow in the vascular access (Qa) for a second dialysis condition, a bolus having the first area content (A) is generated, a first value for the clearance being determined on the basis of an estimate of the clearance for the first dialysis condition and a second value for the clearance being determined on the basis of the change in the physical or chemical parameter in the form of the bolus having the first area content (A) under the second dialysis condition, on the basis of which values the blood flow in the vascular access is determined, wherein,

under the first dialysis condition, the first bloodline (7) conveys blood from a downstream portion (11) of a patient's vascular access (12) and the second bloodline (9) conveys blood towards an upstream portion (13) of the vascular access (11) and, under the second dialysis condition, the first bloodline (7) conveys blood from an upstream portion (13) of the vascular access (12) and the second bloodline (9) conveys blood towards a downstream portion (11) of the vascular access (11).

15. The computer program product according to any of claims 12 to 14, **characterised in that** the second haemodialysis parameter is the dialysis dose Kt/V, where K is the clearance, t is the dialysis time and V is the urea distribution volume, and **in that**, to determine the dialysis dose Kt/V, a bolus having the second area content (A') is generated, a value for the clearance being determined on the basis of the change in the physical or chemical parameter in the form of the bolus having the second area content (A'), on the basis of which value the dialysis dose Kt/V is determined.

**Revendications**

1. Dispositif de dialyse conçu pour raccorder un dialyseur (1) qui est divisé par une membrane semi-perméable (2) en un premier compartiment (3) faisant partie d'un circuit sanguin extracorporel (5) et en un deuxième compartiment (4) faisant partie d'un système de liquide de dialyse (6) du dispositif de dialyse, comprenant
un dispositif (22) destiné à déterminer au moins un premier et un deuxième paramètre d'hémodialyse, le dispositif destiné à déterminer un paramètre d'hémodialyse comprenant :

un moyen (21) destiné à générer une variation dans le temps d'une grandeur caractéristique physique ou chimique d'un liquide de dialyse dans une conduite d'alimentation en liquide de dialyse (17) vers le dialyseur (1) dans le système de liquide de dialyse (6), sous la forme d'un bolus,
un moyen (22) destiné à détecter une variation dans le temps, due au bolus, de la grandeur caractéristique physique ou chimique du liquide de dialyse dans une conduite d'évacuation de liquide de dialyse (18) provenant du dialyseur dans le système de liquide de dialyse (6),
une unité de calcul et d'évaluation (23) qui coopère avec le moyen (21) destiné à générer une variation dans le temps d'une grandeur caractéristique physique ou chimique et avec le moyen (22) destiné à détecter une variation dans le temps d'une grandeur caractéristique physique ou chimique, et qui est configurée de telle sorte qu'au moins une valeur pour la clairance du traitement par dialyse est déterminée sur la base d'une variation de la grandeur caractéristique physique ou chimique, et qu'au moins un paramètre d'hémodialyse est déterminé sur la base de ladite au moins une valeur pour la clairance,
**caractérisé en ce que**
le moyen (21) destiné à générer une variation d'une grandeur caractéristique physique ou chimique est conçu de telle sorte que
pour déterminer un premier paramètre d'hémodialyse, au moins un bolus destiné à déterminer le premier paramètre d'hémodialyse est généré avec une première taille (A), l'unité de calcul et d'évaluation (23) étant configurée de telle sorte que, sur la base de la variation de la grandeur caractéristique physique ou chimique sous la forme d'au moins un bolus destiné à déterminer le premier paramètre d'hémodialyse, au moins une valeur pour la clairance du traitement par dialyse est déterminée, sur la base de laquelle le premier paramètre d'hémodialyse est déterminé, et
pour déterminer un deuxième paramètre d'hémodialyse, au moins un bolus destiné à déterminer le deuxième paramètre d'hémodialyse est généré avec une deuxième taille (A'), l'unité de calcul et d'évaluation (23) étant configurée de telle sorte que, sur la base de la variation de la grandeur caractéristique physique ou chimique sous la forme d'au moins un bolus destiné à déterminer le deuxième paramètre d'hémodialyse, au moins une valeur pour la clairance du traitement par dialyse est déterminée, sur la base de laquelle le deuxième paramètre d'hémodialyse est déterminé,
la première taille (A) étant supérieure à la deuxième taille (A') ou l'écart entre la première taille (A) et une grandeur de référence étant supérieur à l'écart entre la deuxième taille (A') et une grandeur de référence.

2. Dispositif de dialyse selon la revendication 1,
**caractérisé en ce que** ledit au moins un bolus destiné à déterminer le premier paramètre d'hémodialyse est **caractérisé par** une première surface, et ledit au moins un bolus destiné à déterminer le deuxième paramètre d'hémodialyse est **caractérisé par** une deuxième surface, la première aire de la première surface étant supérieure à la deuxième aire de la deuxième surface.

3. Dispositif de dialyse selon la revendication 1 ou 2,
**caractérisé en ce que** le premier paramètre d'hémodialyse est un débit sanguin dans l'accès vasculaire (Qa) ou un débit de recirculation dans un système vasculaire.

4. Dispositif de dialyse selon la revendication 3,

**caractérisé en ce que** le dispositif de dialyse est conçu pour être raccordé à un circuit sanguin extracorporel (5) qui comprend une première conduite de sang (7) et une deuxième conduite de sang (9) qui doivent être raccordées respectivement à une entrée (3a) et à une sortie (3b) du premier compartiment (3) du dialyseur (1), et **en ce que**, pour déterminer le débit sanguin dans l'accès vasculaire (Qa), le moyen (21) destiné à générer une variation d'une grandeur caractéristique physique ou chimique est conçu de telle sorte que, pour une première condition de dialyse, un premier bolus avec la première aire (A) est généré et, pour une deuxième condition de dialyse, un deuxième bolus avec la première aire (A) est généré, l'unité de calcul et d'évaluation (24) étant configurée de telle sorte que, sur la base de la variation de la grandeur caractéristique physique ou chimique sous la forme du premier bolus avec la première aire (A) dans la première condition de dialyse, une première valeur pour la clairance est déterminée et, sur la base de la variation de la grandeur caractéristique physique ou chimique sous la forme du deuxième bolus dans la deuxième condition de dialyse, une deuxième valeur pour la clairance est déterminée, sur la base de laquelle le débit sanguin dans l'accès vasculaire est déterminé,

ou

pour déterminer le débit sanguin dans l'accès vasculaire (Qa), le moyen (21) destiné à générer une variation d'une grandeur caractéristique physique ou chimique est conçu de telle sorte que, pour une deuxième condition de dialyse, un bolus avec la première aire (A) est généré, l'unité de calcul et d'évaluation étant configurée de telle sorte qu'une première valeur pour la clairance est déterminée sur la base d'une estimation de la clairance pour la première condition de dialyse, et sur la base de la variation de la grandeur caractéristique physique ou chimique sous la forme du bolus avec la première aire (A) dans la deuxième condition de dialyse, une deuxième valeur pour la clairance est déterminée, sur la base de laquelle le débit sanguin dans l'accès vasculaire est déterminé, sachant que

dans la première condition de dialyse, la première conduite de sang (7) transporte le sang depuis une partie aval (11) d'un accès vasculaire (12) du patient, et la deuxième conduite de sang (9) transporte le sang vers une partie amont (13) de l'accès vasculaire (11), et dans la deuxième condition de dialyse, la première conduite de sang (7) transporte le sang depuis une partie amont (13) de l'accès vasculaire (12), et la deuxième conduite de sang (9) transporte le sang vers une partie aval (11) de l'accès vasculaire (11).

5. Dispositif de dialyse selon l'une des revendications 1 à 4,
**caractérisé en ce que** le deuxième paramètre d'hémodialyse est la dose de dialyse Kt/V, où K est la clairance, t est la durée de dialyse et V est le volume de distribution de l'urée.

6. Dispositif de dialyse selon la revendication 5,
**caractérisé en ce que**, pour déterminer la dose de dialyse Kt/V, le moyen (21) destiné à générer une variation d'une grandeur caractéristique physique ou chimique est conçu de telle sorte qu'un bolus avec la deuxième aire (A') est généré, l'unité de calcul et d'évaluation (23) étant configurée de telle sorte que, sur la base de la variation de la grandeur caractéristique physique ou chimique sous la forme du bolus avec la deuxième aire (A'), une valeur pour la clairance est déterminée, sur la base de laquelle la dose de dialyse Kt/V est déterminée.

7. Dispositif de dialyse selon l'une des revendications 1 à 6,
**caractérisé en ce qu'**il est prévu une unité de commande et de calcul (24) qui coopère avec le moyen (21) destiné à générer une variation d'une grandeur caractéristique physique ou chimique et avec le moyen (22) destiné à détecter une variation dans le temps d'une grandeur caractéristique physique ou chimique, unité qui est configurée de telle sorte que, pendant le traitement du sang, pour déterminer le premier ou le deuxième paramètre d'hémodialyse, la génération et la détection de la variation de la grandeur caractéristique physique ou chimique sont déclenchées.

8. Dispositif de dialyse selon la revendication 7,

**caractérisé en ce que** l'unité de commande et de calcul (24) est configurée de telle sorte que
pour déterminer le premier paramètre d'hémodialyse, la génération et la détection de la variation de la grandeur caractéristique physique ou chimique pendant le traitement du sang ne sont déclenchées qu'une seule fois ou seulement deux fois,
et/ou
pour déterminer le deuxième paramètre d'hémodialyse, la génération et la détection de la variation de la grandeur caractéristique physique ou chimique pendant le traitement du sang sont déclenchées plusieurs fois.

9. Dispositif de dialyse selon l'une des revendications 4 à 8,
**caractérisé en ce que**

pour déterminer le premier paramètre d'hémodialyse, le moyen (21) destiné à générer une variation d'une grandeur caractéristique physique ou chimique est conçu de telle sorte que la concentration d'une substance dans le liquide de dialyse, en particulier la concentration en Na, est augmentée pour déterminer les première et deuxième valeurs de la clairance dans les première et deuxième conditions de dialyse ou pour déterminer la valeur de la clairance dans la deuxième condition de dialyse, ou est diminuée pour déterminer les première et deuxième valeurs de la clairance dans les première et deuxième conditions de dialyse ou pour déterminer la valeur de la clairance dans la deuxième condition de dialyse,
et/ou
pour déterminer le deuxième paramètre d'hémodialyse, le moyen (21) destiné à générer une variation d'une grandeur caractéristique physique ou chimique est conçu de telle sorte que la concentration d'une substance dans le liquide de dialyse, en particulier la concentration en Na, est augmentée pour une mesure précédente et est diminuée pour une mesure suivante, ou est diminuée pour une mesure précédente et est augmentée pour une mesure suivante.

10. Système de dialyse comprenant un dispositif de dialyse selon l'une des revendications 1 à 9 et un dialyseur qui est divisé par une membrane semi-perméable (2) en un premier compartiment (3) faisant partie d'un circuit sanguin extracorporel (5) et en un deuxième compartiment (4) faisant partie d'un système de liquide de dialyse (6) du dispositif de dialyse, et comprenant un circuit sanguin extracorporel qui comprend une première conduite de sang (7) et une deuxième conduite de sang (9) qui sont reliées respectivement à une entrée (3a) et à une sortie (3b) du premier compartiment (3) du dialyseur (1).

11. Produit de programme informatique comprenant des instructions qui, lors de l'exécution du programme par un ordinateur, amènent celui-ci à mettre en œuvre un procédé pour déterminer au moins un premier et un deuxième paramètre d'hémodialyse pendant un traitement par dialyse avec un dispositif de dialyse, le procédé comprenant les étapes suivantes consistant à :

   générer au moins une variation dans le temps d'une grandeur caractéristique physique ou chimique d'un liquide de dialyse dans une conduite d'alimentation en liquide de dialyse (17) vers un dialyseur (1) dans un système de liquide de dialyse (6), sous la forme d'un bolus,
   détecter au moins une variation dans le temps, due audit au moins un bolus, de la grandeur caractéristique physique ou chimique du liquide de dialyse dans une conduite d'évacuation de liquide de dialyse (18) provenant du dialyseur dans le système de liquide de dialyse (6),
   déterminer au moins une valeur pour la clairance du traitement par dialyse sur la base de ladite au moins une variation de la grandeur caractéristique physique ou chimique sous la forme d'un bolus, et déterminer au moins un paramètre d'hémodialyse sur la base de ladite au moins une valeur pour la clairance,
   dans lequel
   pour déterminer un premier paramètre d'hémodialyse, au moins un bolus destiné à déterminer le premier paramètre d'hémodialyse est généré avec une première taille (A), et, sur la base de la variation de la grandeur caractéristique physique ou chimique sous la forme d'au moins un bolus destiné à déterminer le premier paramètre d'hémodialyse, au moins une valeur pour la clairance du traitement par dialyse est déterminée, sur la base de laquelle le premier paramètre d'hémodialyse est déterminé, et
   pour déterminer un deuxième paramètre d'hémodialyse, au moins un bolus destiné à déterminer le deuxième paramètre d'hémodialyse est généré avec une deuxième taille (A'), et, sur la base de la variation de la grandeur caractéristique physique ou chimique sous la forme d'au moins un bolus destiné à déterminer le deuxième paramètre d'hémodialyse, au moins une valeur pour la clairance du traitement par dialyse est déterminée, sur la base de laquelle le deuxième paramètre d'hémodialyse est déterminé,
   la première taille (A) étant supérieure à la deuxième taille (A') ou l'écart entre la première taille (A) et une grandeur de référence étant supérieur à l'écart entre la deuxième taille (A') et une grandeur de référence.

12. Produit de programme informatique selon la revendication 11,
    dans lequel le procédé est **caractérisé en ce que** ledit au moins un bolus destiné à déterminer le premier paramètre d'hémodialyse est **caractérisé par** une première surface, et ledit au moins un bolus destiné à déterminer le deuxième paramètre d'hémodialyse est **caractérisé par** une deuxième surface, la première aire de la première surface étant supérieure à la deuxième aire de la deuxième surface.

13. Produit de programme informatique selon la revendication 11 ou 12, dans lequel le procédé est **caractérisé en ce que** le premier paramètre d'hémodialyse est un débit sanguin dans l'accès vasculaire (Qa) ou un débit de recirculation dans un système vasculaire.

14. Produit de programme informatique selon la revendication 13,

    dans lequel le procédé est **caractérisé en ce que**
    pour déterminer le débit sanguin dans l'accès vasculaire (Qa) pour une première condition de dialyse, un premier bolus avec la première aire (A) et, pour une deuxième condition de dialyse, un deuxième bolus avec la première aire (A) est généré,
    sur la base de la variation de la grandeur caractéristique physique ou chimique sous la forme du premier bolus avec la première aire (A) dans la première condition de dialyse, une première valeur pour la clairance est déterminée et, sur la base de la variation de la grandeur caractéristique physique ou chimique sous la forme du deuxième bolus dans la deuxième condition de dialyse, une deuxième valeur pour la clairance est déterminée, sur la base de laquelle le débit sanguin dans l'accès vasculaire est déterminé,

ou

pour déterminer le débit sanguin dans l'accès vasculaire (Qa) pour une deuxième condition de dialyse, un bolus avec la première aire (A) est généré, une première valeur pour la clairance étant déterminée sur la base d'une estimation de la clairance pour la première condition de dialyse, et sur la base de la variation de la grandeur physique ou chimique sous la forme du bolus avec la première aire (A) dans la deuxième condition de dialyse, une deuxième valeur pour la clairance est déterminée, sur la base de laquelle le débit sanguin dans l'accès vasculaire est déterminé,

sachant que

dans la première condition de dialyse, la première conduite de sang (7) transporte le sang depuis une partie aval (11) d'un accès vasculaire (12) du patient, et la deuxième conduite de sang (9) transporte le sang vers une partie amont (13) de l'accès vasculaire (11), et dans la deuxième condition de dialyse, la première conduite de sang (7) transporte le sang depuis une partie amont (13) de l'accès vasculaire (12) et la deuxième conduite de sang (9) transporte le sang vers une partie aval (11) de l'accès vasculaire (11).

**15.** Produit de programme informatique selon l'une des revendications 12 à 14,

**caractérisé en ce que** le deuxième paramètre d'hémodialyse est la dose de dialyse Kt/V, où K est la clairance, t est la durée de dialyse et V est le volume de distribution de l'urée,

et **en ce que**, pour déterminer la dose de dialyse Kt/V, un bolus avec la deuxième aire (A') est généré, et, sur la base de la variation de la grandeur caractéristique physique ou chimique sous la forme du bolus avec la deuxième aire (A'), une valeur pour la clairance est déterminée sur la base de laquelle la dose de dialyse Kt/V est déterminée.

Fig. 1

EP 4 142 822 B1

# Fig. 2A

# Fig. 2B

Fig. 3

Fig. 4

Fig. 5

Fig. 6

Fig. 7

Fig. 8

Fig. 9

Fig. 10

# EP 4 142 822 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0911043 B1
- EP 0928614 B1
- US 20050148923 A1
- EP 1698360 B1
- DE 3223051 A1

### In der Beschreibung aufgeführte Nicht-Patentliteratur

- Principles and biophysics of Dialysis. **SARGENT, A.** ; **GOTCH, F.** Replacement of renewal function by dialysis
- **SARGENT, A.** ; **GOTCH, F.** Principles and biophysics of Dialysis'' in Replacement of renewal function by dialysis